# EUROPEAN PATENT APPLICATION

(11) **EP 4 624 470 A1**
(43) Date of publication of application: **01.10.2025**
(21) Application number: 23893630.6
(22) Date of filing: 07.11.2023
(51) Int. Cl.: C07D 459/00, A61K 31/475, A61K 31/551, A61P 3/10, A61P 7/02

(54) **YOHIMBINE DERIVATIVE, AND PREPARATION METHOD THEREFOR AND PHARMACEUTICAL COMPOSITION AND USE THEREOF**

(30) Priority: 21.11.2022 CN 202211462324
(71) Applicant: Shanghai Institute of Materia Medica, Chinese Academy of Sciences, Shanghai 201203 (CN); Shenzhen Institutes of Advanced Technology Chinese Academy of Sciences, Shenzhen, Guangdong 518055 (CN)
(72) Inventor: NAN, Fajun, Shanghai 201203 (CN); CHEN, Zuxin, Shenzhen, Guangdong 518055 (CN); XIE, Xin, Shanghai 201203 (CN); WANG, Jingyi, Shanghai 201203 (CN); LIU, Xinan, Shenzhen, Guangdong 518055 (CN); GUO, Shimeng, Shanghai 201203 (CN); CHEN, Dongsheng, Shanghai 201203 (CN); HONG, Feng, Shenzhen, Guangdong 518055 (CN); XIE, Yinfang, Shenzhen, Guangdong 518055 (CN); FANG, Jiahui, Shanghai 201203 (CN)
(74) Representative: Lavoix
(86) International application number: PCT/CN2023/130285
(87) International publication number: WO 2024/109539

(57) **Abstract**

A yohimbine derivative, and a preparation method therefor and a pharmaceutical composition and use thereof. The biological structure of the yohimbine derivative is as shown by formula (I), wherein the definitions of the substituents in the formula are as stated in the description and claims. The yohimbine derivative is used as an α2A-AR antagonist, and can be used for treating diseases such as diabetes.

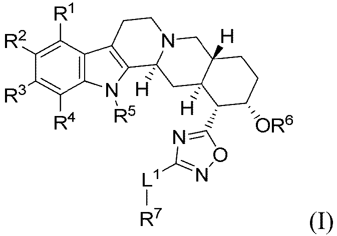

## Description

### Technical field

The present invention relates to the field of biomedicine, and in particular to a class of α2A-AR antagonists, more specifically, to a class of yohimbine derivatives, and a preparation method thereof, as well as a pharmaceutical composition containing the compound, the use of the compound in the preparation of the α2A-AR antagonist, and the use of the compound in the preparation of a drug for treating diabetes.

### Background technique

The alpha2-adrenergic receptor (α2-AR) belongs to the G protein-coupled receptor (GPCR) superfamily and is very important for regulating catecholamine signaling. In pharmacology, a2-AR is divided into three subtypes: α2A-AR, α2B-AR, and α2C-AR. Among them, α2A-AR is widely distributed in the central nervous system (CNS) and peripheral tissues, and the former is predominant, accounting for about 90% of α2-AR in the CNS. α2A-AR can inhibit neuronal excitation and the release of norepinephrine and other neurotransmitters, thereby mediating a series of important physiological responses and pharmacological effects. Multiple polymorphisms of the α2A-AR encoding gene ADRA2A have been identified, which can increase the expression of α2A-AR, reduce antidepressant responses, and change memory and behavior in different ways.

Studies have shown that a genetic variant of the ADRA2A gene is closely related to type 2 diabetes (T₂D): the excess α2A-AR produced in pancreatic β cells of carriers of the risk allele rs553668 inhibits the docking of insulin granules with the plasma membrane, resulting in a decrease in the arrangement of insulin-containing vesicles on the cell membrane, thereby affecting the level of insulin release (Science, 2010, 327, 217-220; N. Engl. J. Med., 2010, 362, 361-362). Subsequent studies have confirmed that the α2A-AR antagonist yohimbine can significantly improve the insulin secretion defect associated with the ADRA2A risk variable in patients (Sci. Transl. Med., 2014, 6, 257ra139). Given that reduced pancreatic β-cell secretory capacity is one of the main characteristics of T₂D, blocking α2A-AR signaling may be a novel therapeutic approach specifically targeting pancreatic β-cell defects in 40% of T₂D patients carrying the rs553668 risk variant.

Yohimbine is clinically used to treat male erectile dysfunction. Its pharmacokinetics have been well characterized. Common side effects include dizziness, anxiety, irritability, insomnia, hypertension and palpitations, which are mainly caused by presynaptic α2A-AR blockade and sympathetic nerve activation in the CNS (EFSA. J., 2013, 11, 3302). Although yohimbine is effective in correcting insulin secretion defects, its common central side effects make it unsuitable as a candidate drug for T₂D treatment, so it needs to be structurally modified and optimized.

At present, there are few reports on the structural modification of yohimbine, which mainly focuses on converting the ester group at position 16 into an amide and examining the binding affinity of the derivatives to the α2-AR subtype (J. Pharmacol. Exp. Ther., 2002, 303, 979-984; Bioorg. Med. Chem. Lett., 2005, 15, 2758-2760; J. Pharmacol. Exp. Ther., 2006, 319, 739-748). So far, there have been no reports on the antagonistic activity and tissue distribution of new yohimbine derivatives on α2A-AR.

### Summary of the invention

The object of the present invention is to provide a yohimbine derivative used as an α2A adrenergic receptor (α2A-AR) antagonist.

The first aspect of the present invention provides a compound represented by formula (I), a deuterated compound thereof or a pharmaceutically acceptable salt thereof, 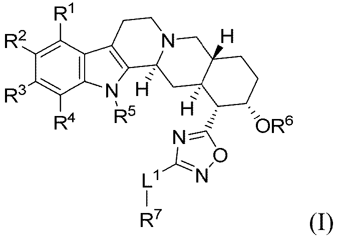
wherein, R¹, R², R³ and R⁴ are each independently hydrogen, halogen, cyano, hydroxyl, ethynyl, substituted or unsubstituted following group: C1-C6 alkyl, C3-C10 cycloalkyl, 4-7 membered heterocycloalkyl containing 1 to 3 heteroatoms, C6-C10 aryl, 5-7 membered heteroaryl, 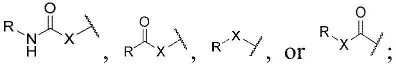 wherein R is the following group: C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{a}; wherein R^{a} is selected from: hydrogen, C1-C6 alkyl, or C3-C10 cycloalkyl;
R⁵ and R⁶ are each independently hydrogen, substituted or unsubstituted following group: C1-C6 alkyl, C3-C10 cycloalkyl, 4-7 membered heterocycloalkyl containing 1 to 3 heteroatoms, 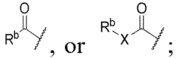 wherein R^{b} is selected from: C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{c}; wherein R^{c} is selected from: hydrogen, C1-C6 alkyl, or C3-C10 cycloalkyl;
L¹ is absent, hydrogen, substituted or unsubstituted following group: C1-C6 alkylene, C3-C10 cycloalkyl, 4-7 membered heterocycloalkyl containing 1 to 3 heteroatoms, C6-C10 aryl, 5-7 membered heteroaryl, 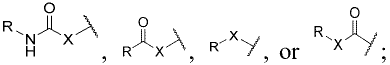 wherein R is a C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{a}; wherein R^{a} is selected from: hydrogen, C1-C6 alkyl, or C3-C10 cycloalkyl;
R⁷ is absent, hydrogen, substituted or unsubstituted following group: C1-C6 alkoxy, C3-C10 cycloalkyl, C6-C10 aryl, 3-7 membered heterocyclyl, 5-7 membered heteroaryl, 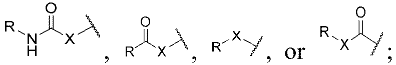 wherein R is a C1-C6 alkyl, C3-C10 cycloalkyl, 4-7 membered heterocyclyl containing 1 to 3 heteroatoms, C6-C10 aryl, or 5-7 membered heteroaryl, X is O or NR^{a}; wherein R^{a} is hydrogen, C1-C6 alkyl, or C3-C10 cycloalkyl;
the substituents separated by 0 to 2 atoms on L¹, R⁷ can be cyclized to form a 3- to 7-membered cycloalkyl ring, a 4- to 7-membered heterocycloalkyl ring, a 6-membered aromatic ring, or a 5- to 7-membered heteroaromatic ring;
the above substitution means that a hydrogen on the group is substituted by one or more substituents selected from a group consisting of halogen, hydroxyl, cyano, C1-C6 haloalkyl (such as trifluoromethyl), alkynyl, C1-C6 alkyl, C1-C6 alkoxy, C1-C6 haloalkoxy (such as trifluoromethoxy), NR^{d}R^{e}, 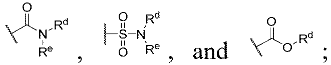 wherein R^{d} and R^{e} are each independently hydrogen or C1-C6 alkyl.

In another preferred embodiment, R¹ is hydrogen, halogen, cyano, hydroxyl, ethynyl, or C1-C6 alkyl;
R² is hydrogen, halogen, cyano, hydroxyl, ethynyl, C1-C4 alkyl, or 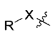 ; wherein R is a substituted or unsubstituted following group: C1-C4 alkyl, X is O, NR^{a}; wherein R^{a} is hydrogen, or C1-C4 alkyl, wherein the substitution means that a hydrogen on the group is substituted by one or more substituents selected from a group consisting of halogen, hydroxyl, C1-C4 alkyl, and NR^{d}R^{e}, wherein R^{d} and R^{e} are each independently hydrogen or C1-C4 alkyl;
R³ is hydrogen, halogen, cyano, hydroxyl, C1-C4 alkyl, or 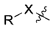 ; wherein R is a substituted or unsubstituted following group: C1-C4 alkyl, X is O, wherein the substitution means that a hydrogen on the group is substituted by one or more substituents selected from a group consisting of halogen, hydroxyl, cyano, C1-C6 alkyl, and NR^{d}R^{e}, wherein R^{d} and R^{e} are each independently hydrogen, or C1-C4 alkyl;
R⁴ is hydrogen, halogen, cyano, or hydroxyl.

In another preferred embodiment, R¹ is hydrogen or fluorine.

In another preferred embodiment, R² is hydrogen, fluorine, chlorine, bromine, cyano, hydroxyl, ethynyl, methyl, or methoxy.

In another preferred embodiment, R³ is hydrogen, fluorine, hydroxyl, ethynyl, methyl, or methoxy.

In another preferred embodiment, R⁴ is hydrogen.

In another preferred embodiment, R⁵ is hydrogen, C1-C4 alkyl or 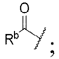 wherein R^{b} is a substituted or unsubstituted following group: C1-C4 alkyl, C3-C6 cycloalkyl, C6-C10 aryl, or 5-7 membered heteroaryl, wherein the substitution means that a hydrogen on the group is substituted by one or more substituents selected from a group consisting of halogen, hydroxyl, C1-C4 alkyl, C1-C4 alkoxy, and NR^{d}R^{e}, wherein R^{d} and R^{e} are each independently hydrogen or C1-C4 alkyl;
R⁶ is hydrogen, C1-C4 alkyl, C3-C6 cycloalkyl, or 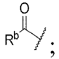 wherein R^{b} is a substituted or unsubstituted following group: C1-C4 alkyl, wherein the substitution means that a hydrogen on the group is substituted by one or more substituents selected from a group consisting of: halogen, hydroxyl, cyano, trifluoromethyl, and NR^{d}R^{e}, wherein R^{d} and R^{e} are each independently hydrogen, or C1-C4 alkyl.

In another preferred embodiment, R⁵ is hydrogen, C1-C3 alkyl, or acetyl.

In another preferred embodiment, R⁶ is hydrogen, C1-C3 alkyl, or acetyl.

In another preferred embodiment, L¹ is absent, hydrogen, substituted or unsubstituted following group: C1-C4 alkylene, C3-C8 cycloalkyl, 5-7 membered heterocycloalkyl containing 1 to 3 heteroatoms, C6-C10 aryl, 5-7 membered heteroaryl, 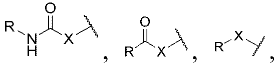 or 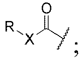 wherein R is the following group: C1-C4 alkyl, C3-C6 cycloalkyl, 4-7 membered heterocycloalkyl containing 1 to 3 heteroatoms, C6-C10 aryl, or 5-7 membered heteroaryl, X is O or NR^{a}; wherein R^{a} is hydrogen, C1-C4 alkyl, or C3-C6 cycloalkyl, wherein the substitution means that a hydrogen on the group is substituted by one or more substituents selected from a group consisting of: halogen, hydroxyl, cyano, trifluoromethyl, C1-C4 alkyl, C1-C4 alkoxy, and NR^{d}R^{e}, wherein R^{d} and R^{e} are each independently hydrogen or C1-C4 alkyl.

In another preferred embodiment, L¹ is absent, hydrogen, substituted or unsubstituted following group: C1-C4 alkylene, C3-C6 cycloalkyl, or 5-7 membered heterocycloalkyl containing 1 to 3 heteroatoms; wherein the substitution means that the hydrogen on the group is substituted by one or more substituents selected from a group consisting of: halogen, hydroxyl, cyano, trifluoromethyl, C1-C4 alkyl, C1-C4 alkoxy, and NR^{d}R^{e}, wherein R^{d} and R^{e} are each independently hydrogen or C1-C4 alkyl.

In another preferred embodiment, R⁷ is absent, hydrogen, substituted or unsubstituted following group: C1-C4 alkoxy, C3-C6 cycloalkyl, C6-C10 aryl, 5-7 membered heterocyclyl, 5-7 membered heteroaryl, 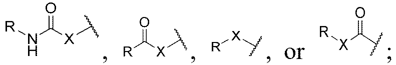 wherein R is the following group: C1-C6 alkyl, C3-C10 cycloalkyl, 4-7 membered heterocyclyl containing 1 to 3 heteroatoms, C6-C10 aryl, or 5-7 membered heteroaryl, X is O or NR^{a}; wherein R^{a} is hydrogen, C1-C6 alkyl, or C3-C10 cycloalkyl;
the substituents separated by 0 to 2 atoms on R⁷ can be cyclized to form a 3- to 7-membered cycloalkyl ring, a 4- to 7-membered heterocycloalkyl ring, a 6-membered aromatic ring, or a 5-to 7-membered heteroaromatic ring.
the substitution means that a hydrogen on the group is substituted by one or more substituents selected from a group consisting of: halogen, hydroxyl, cyano, C1-C4 haloalkyl (such as trifluoromethyl), C1-C4 alkyl, C1-C4 alkoxy, C1-C4 haloalkoxy (such as trifluoromethoxy), NR^{d}R^{e}, 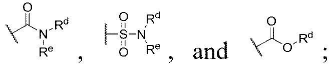 wherein R^{d} and R^{e} are each independently hydrogen or C1-C4 alkyl.

In another preferred embodiment, R⁷ is absent, hydrogen, substituted or unsubstituted following group: C1-C4 alkoxy, C3-C6 cycloalkyl, phenyl, 5-7 membered heterocyclyl, or 5-7 membered heteroaryl;
wherein the substitution means that a hydrogen on the group is substituted by one or more substituents selected from a group consisting of fluorine, chlorine, bromine, hydroxyl, cyano, trifluoromethyl, trifluoromethoxy, alkynyl, C1-C4 alkyl, C1-C4 alkoxy, 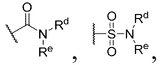 and 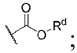 wherein R^{d} and R^{e} are each independently hydrogen or C1-C4 alkyl;
the substituents separated by 0 to 2 atoms on R⁷ can be cyclized to form a 6-membered aromatic ring or a 5-7 membered heteroaromatic ring.

In another preferred embodiment, L¹ and R⁷ form the following formula V: 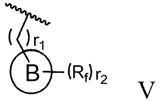
wherein r₁ is selected from 1, 2 or 3; r₂ is selected from 0, 1, 2 or 3;
ring B is phenyl, 5-6 membered heterocyclyl, C3-C6 cycloalkyl, 5-6 membered heteroaryl;
each R_{f} is independently selected from: halogen, hydroxyl, cyano, C1-C4 haloalkyl, C1-C4 alkyl, C1-C4 alkoxy, C1-C4 haloalkoxy, NR^{d}R^{e}, 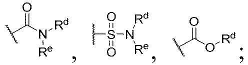 wherein R^{d} and R^{e} are each independently hydrogen, C1-C4 alkyl; or
two adjacent R_{f} and the adjacent C on ring B together form a benzene ring or a 5- to 7-membered heteroaromatic ring.

In another preferred embodiment, ring B is a benzene ring, and the substituent thereon is located at the para position of L¹.

In another preferred embodiment, the compound of formula V has the following structure: 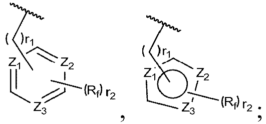
wherein r₁ is selected from 1, 2 or 3; r₂ is selected from 0, 1, 2 or 3;
Z₁, Z₂, Z₃ are each independently selected from O, S, N, CH;
each R_{f} is independently selected from: halogen, hydroxyl, cyano, C1-C4 haloalkyl, C1-C4 alkyl, C1-C4 alkoxy, C1-C4 haloalkoxy, NR^{d}R^{e}, 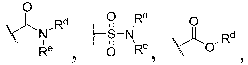 wherein R^{d} and R^{e} are each independently hydrogen, or C1-C4 alkyl; or
two adjacent R_{f} and adjacent C on the ring form a benzene ring or a 5-7 membered heteroaromatic ring. In another preferred embodiment, r₁ is selected from 1 or 2.

In another preferred embodiment, r₂ is selected from 1 or 2.

In another preferred embodiment, Z₁, Z₂, Z₃ are each independently selected from O, N, and CH.

In another preferred embodiment, each R_{f} is independently selected from: fluorine, chlorine, bromine, hydroxyl, cyano, trifluoromethyl, trifluoromethoxy, methyl, ethyl, methoxy, ethoxy, NR^{d}R^{e}, 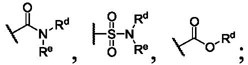 wherein R^{d} and R^{e} are each independently hydrogen, methyl, ethyl; or
two adjacent R_{f} and the adjacent C on the ring together form a benzene ring.

In another preferred embodiment, -L¹-R⁷ is selected from: 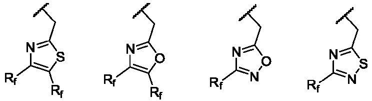 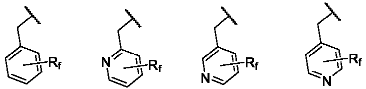 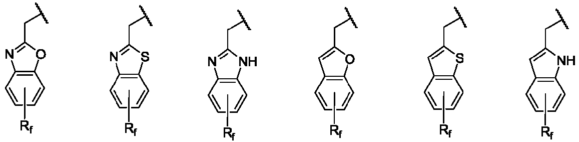 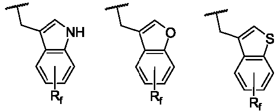 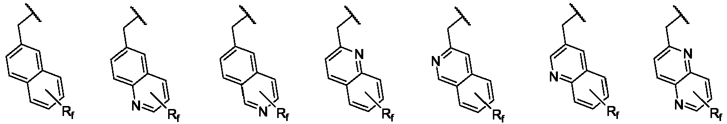 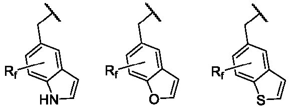 the number of substituents R_{f} on the benzene ring or benzoheteroaromatic ring is 0, 1, 2 or 3, preferably 0, 1 or 2.

In another preferred embodiment, -L¹-R⁷ is selected from: -CH₃,

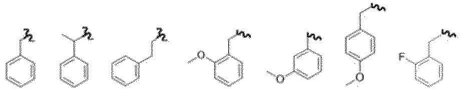

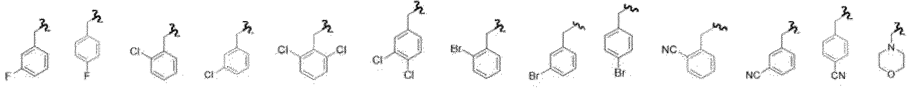

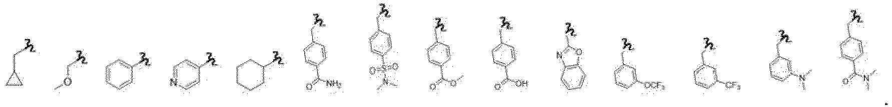

In another preferred embodiment, the compound is selected from C1-C35.

The second aspect of the present invention provides a method for preparing the compound described in the first aspect, wherein 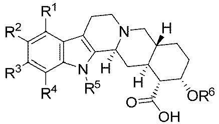 is used as a raw material to obtain the compound by converting the 16-carboxyl group into an oxadiazole structure, wherein the definition of each substituent is as described above.

In a preferred embodiment, the compound or a pharmaceutically acceptable salt thereof is prepared by the following route: 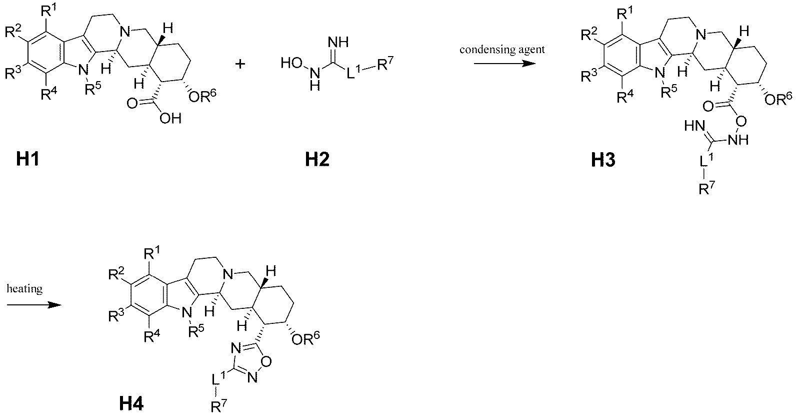
reacting a compound of formula H1 with a compound of formula H2 under the condition of a condensing agent to obtain a compound of formula H3;
subjecting a compound of formula H3 an intramolecular condensation cyclization under heating conditions to form a compound of formula H4;
when L¹ is a methylene group and R⁷ is an iodine-substituted phenyl group, the compound of formula H4 is subjected to a coupling reaction under palladium catalysis to obtain a compound in which L¹ is a methylene group and R⁷ is a cyano-substituted phenyl group;
when L¹ is a methylene group and R⁷ is a phenyl group substituted with a cyano group, the compound is oxidized by hydrogen peroxide under alkaline conditions to obtain a compound in which L¹ is a methylene group and R⁷ is a phenyl group substituted with 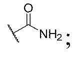
when L¹ is a methylene group and R⁷ is a phenyl group substituted with a methyl ester, the compound of formula H4 is subjected to a hydrolysis reaction to obtain a compound in which L¹ is a methylene group and R⁷ is a carboxyl group substituted phenyl;
the definitions of the substituents are as described above.

The third aspect of the present invention provides a pharmaceutical composition comprising: the compound represented by the general formula (I) described in the first aspect, or a pharmaceutically acceptable salt thereof; and a pharmaceutically acceptable carrier.

The fourth aspect of the present invention provides use of the compound represented by the general formula (I) described in the first aspect or the pharmaceutical composition described in the third aspect, (i) for preparing an α2A adrenergic receptor (α2A-AR) antagonist; or (ii) for preparing a drug for treating diabetes.

The fifth aspect of the present invention provides a method for treating diabetes, comprising administering the compound described in the first aspect or a pharmaceutically acceptable salt thereof, or the pharmaceutical composition described in the third aspect to a subject in need thereof.

The novel oxadiazole yohimbine derivatives of the present invention can antagonize α2a adrenaline receptor at a micromolar concentration, and has the characteristics of pancreas targeting and significantly lower brain tissue distribution characteristics compared with yohimbine.

It should be understood that within the scope of the present invention, the above-mentioned technical features of the present invention and the technical features specifically described below (such as examples) can be combined with each other to form a new or preferred technical solution. Each feature disclosed in the specification can be replaced by any alternative feature that provides the same, equal or similar purpose. Due to space limitations, they will not be described one by one here.

### Brief description of the drawings

Figure 1 shows the tissue concentrations (ng/g or ng/mL) of different compounds in mice 2 hours after gavage of 10 mg/kg of different compounds.
Figure 2 shows the tissue concentrations (ng/g or ng/mL) of compound C27 in mice 0.25, 2, and 8 hours after gavage of 10 mg/kg of compound C27.
Figure 3 shows the basal blood glucose level of type 2 diabetic mice after a single intraperitoneal injection of yohimbine derivatives (10 mg/kg).
Figure 4 shows the glucose tolerance results of type 2 diabetic mice after a single intraperitoneal injection of yohimbine derivatives (10 mg/kg).
Figure 5 shows the results of insulin sensitivity in type 2 diabetic mice after a single intraperitoneal injection of yohimbine derivatives (10 mg/kg).
Figure 6 shows the glucose tolerance results of type 2 diabetic mice after a single gavage administration of yohimbine derivatives (10 mg/kg).
Figure 7 shows the results of insulin sensitivity in type 2 diabetic mice after a single gavage administration of yohimbine derivatives (10 mg/kg).
Figure 8 shows the basal blood glucose level of type 2 diabetic mice chronically injected with yohimbine derivatives (10 mg/kg, once a day).

### Detailed description of the invention

After extensive and in-depth research, the inventors of the present application have developed for the first time a class of compounds that have both pancreatic targeting and low brain permeability while maintaining good α2A adrenergic receptor antagonist activity. Specifically, it is a class of oxadiazole yohimbine derivatives, whose main feature is that the ester group at the 16th position of yohimbine is converted into an oxadiazole group, which can maintain good α2A adrenergic receptor antagonist activity. Compared with yohimbine, this class of derivatives has the characteristics of pancreatic targeting and significantly reduced brain tissue distribution characteristics relative to yohimbine, eliminates central side effects such as anxiety and hypertension caused by yohimbine, can significantly improve blood sugar metabolism in animal models of type 2 diabetes, and is expected to become a new drug for treating diabetes that acts on α2A adrenergic receptors. On this basis, the present invention is completed.

### Term

Herein, the alkyl is preferably an aliphatic alkyl , which may be a linear alkyl or a branched alkyl, including but not limited to: methyl, ethyl, n-propyl, isopropyl, n-butyl, isobutyl, tert-butyl, etc.; expressions such as "C1-C6" are intended to include corresponding groups having 1, 2, 3, 4, 5 or 6 carbon atoms, for example, "C1-C6 alkyl" refers to an alkyl having 1, 2, 3, 4, 5 or 6 carbon atoms.

"Alkylene" refers to a straight or branched saturated aliphatic group having a specified number of carbon atoms and connecting at least two other groups, i.e., a divalent hydrocarbon group. The two groups connected to the alkylene group can be connected to the same or different atoms on the alkylene group. For example, a straight chain alkylene group can be a divalent group of -(CH₂)ₙ-, wherein n is 1, 2, 3, 4, 5 or 6. Representative alkylene groups include, but are not limited to, methylene, ethylene, propylene, isopropylene, butylene, isobutylene, sec-butylene, pentylene and hexylene.

Herein, the halogen preferably refers to fluorine, chlorine, bromine or iodine.

Herein, the alkoxy refers to -O-(alkyl), wherein the alkyl is as defined above. "C1-C6 alkoxy" refers to an oxygen-containing alkyl containing 1 to 6 carbon atoms, and non-limiting examples include methoxy, ethoxy, propoxy, butoxy, and the like.

Herein, the cycloalkyl can be a saturated or partially unsaturated monocyclic or polycyclic hydrocarbon substituent, including 3 to 20 carbon atoms, preferably including 3 to 12 carbon atoms, more preferably 3 to 10 carbon atoms. Non-limiting examples of monocyclic cycloalkyl include cyclopropyl, cyclobutyl, cyclopentenyl, cyclohexyl, cyclooctyl, etc.; polycyclic cycloalkyl includes spiro-, fused and bridged cycloalkyl.

Herein, the aryl refers to a 6-10-membered all-carbon monocyclic or fused polycyclic (i.e., a ring that shares adjacent carbon atoms) group, and the group has a conjugated π electron system, such as phenyl and naphthyl. The aryl ring can be fused with a heterocyclyl, heteroaryl or cycloalkyl, and non-limiting examples include benzimidazole, benzothiazole, benzoxazole, benzisoxazole, benzopyrazole, quinoline, benzindole, and benzodihydrofuran.

Herein, the heterocyclyl refers to an alicyclic heterocyclic system containing 1 to 3 heteroatoms, such as 3 to 7 ring atoms. The heteroatoms of the heterocyclyl include oxygen, sulfur and nitrogen. The heterocyclyl is preferably 3-membered or 6-membered, such as oxiranyl, morpholinyl, piperazinyl, etc.

Herein, the heteroaryl refers to a heteroaromatic system containing 1 to 4 heteroatoms, such as 5 to 14 ring atoms. The heteroatoms of the heteroaryl include oxygen, sulfur and nitrogen. The heteroaryl is preferably a 5- or 6-membered group, such as furyl, thienyl, pyridyl, pyrrolyl, N-alkylpyrrolyl, pyrimidyl, pyrazinyl, imidazolyl, tetrazolyl, etc. The heteroaryl can be fused to an aryl, heterocyclyl or cycloalkyl ring, wherein the ring connected to the parent structure is a heteroaryl ring.

Unless otherwise indicated in the present invention, 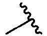 represents a linking site.

Herein, the pharmaceutically acceptable salts are not particularly limited, and preferably include: inorganic acid salts, organic acid salts, alkyl sulfonates and aryl sulfonates; the inorganic salts include hydrochlorides, hydrobromides, nitrates, sulfates, phosphates, etc.; the organic salts include formates, acetates, propionates, benzoates, maleates, fumarates, succinates, tartrates, citrates, etc.; the alkyl sulfonates include methyl sulfonates, ethyl sulfonates, etc.; the aryl sulfonates include benzene sulfonates, p-toluene sulfonates, etc.

### Preparation method

The oxadiazole yohimbine derivatives of the present invention can be prepared by the following route: The definitions of the substituents are the same as those described above.

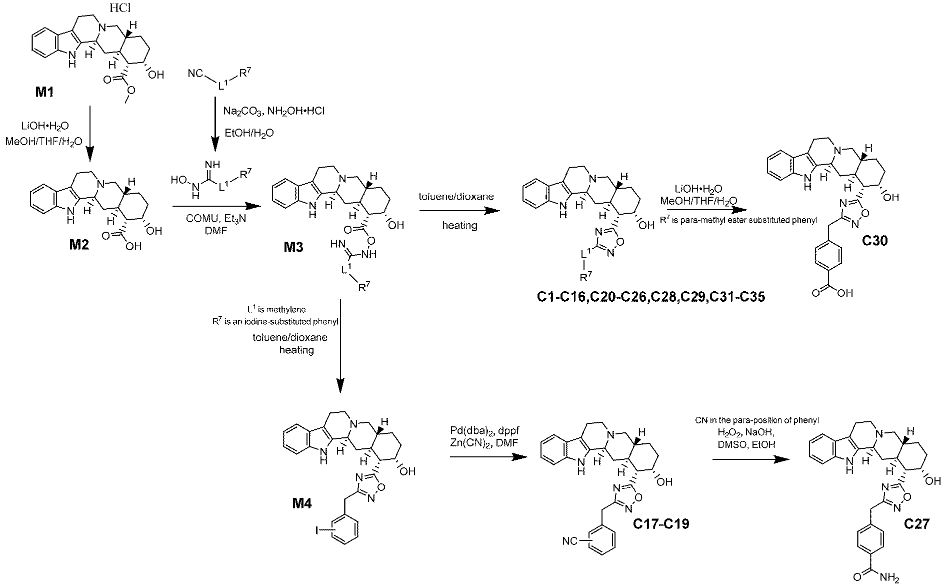

The present invention will be further described below in conjunction with specific examples. It should be understood that these examples are intended only to illustrate the present invention and are not intended to limit the scope of the present invention. The experimental methods for the unrecorded specific conditions in the following examples are usually performed under normal conditions or according to the conditions recommended by the manufacturer. Unless otherwise indicated, percentages and parts are weight percentages and weight parts.

Unless otherwise defined, all professional and scientific terms used herein have the same meanings as those familiar to those skilled in the art. In addition, any methods and materials similar or equivalent to those described herein can be applied to the methods of the present invention. The preferred implementation methods and materials described herein are for demonstration purposes only.

In the following preparation examples, NMR was measured using a Bruker Avance III 400/500 MHz NMR instrument, and NMR calibration was: δH 7.26 ppm (CDCl₃), 2.50 ppm (DMSO-*d₆*); mass spectrometry was measured using an Agilent 1200 Quadrupole LC/MS liquid chromatography-mass spectrometer; reagents were mainly provided by Shanghai Chemical Reagent Company; TLC thin layer chromatography silica gel plates were produced by Shandong Yantai Jiangyou Silica Gel Development Co., Ltd., model HSGF 254; normal phase column chromatography silica gel used for compound purification was produced by Shandong Qingdao Ocean Chemical Plant Branch, model zcx-II, 200-300 mesh.

The Chinese equivalents of the abbreviations herein are as follows:
DMF: N,N-dimethylformamide; COMU: (1-cyano-2-ethoxy-2-oxyethyleneaminooxy) dimethylamino-morpholinyl-carbonium hexafluorophosphate;
Pd(dba)₂: bis(dibenzylideneacetonepalladium); dppf: 1,1'-bis(diphenylphosphino)ferrocene.

### Example 1

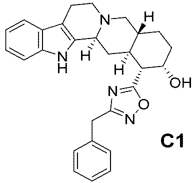

(1) At room temperature, benzyl cyanide (1.17 g, 10 mmol) was dissolved in ethanol (20 mL), and water (8 mL), sodium carbonate (848 mg, 8 mmol), and hydroxylamine hydrochloride (695 mg, 10 mmol) were added in sequence. After the addition, the mixture was stirred at room temperature for 24 hours, and most of the solvent was removed by concentration under reduced pressure. The residue was dissolved in ethyl acetate (80 mL), washed with saturated brine (20 mL × 2), and the organic phase was dried over anhydrous sodium sulfate and concentrated under reduced pressure. The product was directly used for condensation reaction without purification.
(2) Yohimbine hydrochloride M1 (7.81 g, 20 mmol) was placed in methanol/tetrahydrofuran/water (90 mL/60 mL/30 mL) under ice bath, and lithium hydroxide monohydrate (5.04 g, 120 mmol) was added. After stirring under ice bath for half an hour, the mixture was warmed to room temperature and monitored by LC-MS until the starting material disappeared. Most of the solvent was removed by concentration under reduced pressure, and the residue was dissolved in water (150 mL). The pH was adjusted with 5% hydrochloric acid under ice bath until a milky white turbidity appeared in the system. The mixture was allowed to stand overnight and filtered. The filter cake was washed with water (100 mL). The filter cake was collected and dried to obtain white powder M2 (5.9 g, 17.3 mmol) with a molar yield of 86%.
(3) At room temperature, M2 (340 mg, 1 mmol) was dissolved in DMF (5 mL), triethylamine (304 mg, 3 mmol) was added, and COMU (428 mg, 1 mmol) was slowly added after stirring for 5 minutes. After the addition was completed, the mixture was stirred at room temperature for 20 minutes, and the crude product (300 mg, 2 mmol) in operation (1) was added. After stirring at room temperature for 24 hours, the reaction solution was concentrated under reduced pressure to remove the solvent. The residue was dissolved in dichloromethane/methanol (100 mL/10 mL), washed with saturated brine (20 mL×3), and the organic phase was dried over anhydrous sodium sulfate, concentrated under reduced pressure, and purified by column chromatography (dichloromethane/methanol = 30:1) to obtain a brown foamy solid M3.
(4) The condensation product M3 in operation (3) was dissolved in 1,4-dioxane (5 mL), and toluene (10 mL) was added. The mixture was heated to 100 °C under a nitrogen atmosphere. The reaction was stopped after 24 hours. The solvent was removed by concentration under reduced pressure. The residue was dissolved in dichloromethane/methanol (100 mL/10 mL), washed with saturated brine (20 mL×3), and the organic phase was dried over anhydrous sodium sulfate, concentrated under reduced pressure, and purified by column chromatography (dichloromethane/methanol=30:1) to obtain a light brown foamy solid C1 (46 mg, 0.1 mmol). The molar yield of operations (3) and (4) was 10%.

The other compounds in the following table were synthesized using the same method as in Example 1 with different cyano substrates:

| compound | structure | ¹H-NMR |
|---|---|---|
| **C1** | 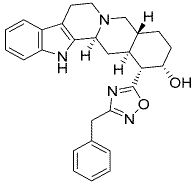 | ¹H-NMR (CDCl₃, 400 MHz): δ (ppm) 7.62 (s, 1H), 7.48-7.29 (m, 6H), 7.17-7.08 (m, 2H), 4.24 (s, 1H), 4.17 (s, 2H), 3.57 (s, 1H), 3.30 (d, *J* = 11.2 Hz, 1H), 3.14-3.10 (m, 1H), 3.02-2.91 (m, 3H), 2.76-2.64 (m, 2H), 2.30-2.25 (m, 1H), 2.14-2.09 (m, 2H), 1.73-1.62 (m, 5H), 1.52-1.42 (m, 2H) |
| **C2** | 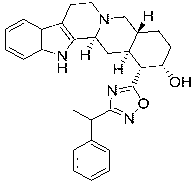 | ¹H-NMR (CDCl₃, 400 MHz): δ (ppm) 7.57 (d, *J* = 14.0 Hz, 1H), 7.48-7.28 (m, 7H), 7.18-7.09 (m, 2H), 4.40-4.34 (m, 1H), 4.24 (d, *J* = 6.0 Hz, 1H), 3.68-3.60 (m, 1H), 3.27 (d, *J* = 11.2 Hz, 1H), 3.12-3.10 (m, 1H), 3.04-2.90 (m, 3H), 2.75-2.71 (m, 1H), 2.66-2.60 (m, 1H), 2.29-2.24 (m, 1H), 2.13-2.08 (m, 2H), 1.80 (dd, *J* = 7.2 Hz, 3.2 Hz, 3H), 1.71-1.65 (m, 4H), 1.52-1.40 (m, 2H) |
| **C3** | 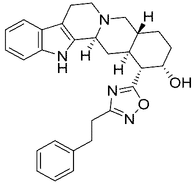 | ¹H-NMR (CDCl₃, 400 MHz): δ (ppm) 7.59 (s, 1H), 7.48 (d, *J* = 7.6 Hz, 1H), 7.36-7.24 (m, 6H), 7.18-7.09 (m, 2H), 4.22 (s, 1H), 3.82 (s, 1H), 3.29 (d, *J* = 10.8 Hz, 1H), 3.18-3.10 (m, 5H), 3.02-2.92 (m, 3H), 2.76-2.72 (m, 1H), 2.68-2.63 (m, 1H), 2.32-2.26 (m, 1H), 2.14-2.12 (m, 2H), 1.70-1.63 (m, 4H), 1.56-1.42 (m, 2H) |
| **C4** | 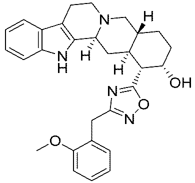 | ¹H-NMR (DMSO-*d₆*, 400 MHz): δ (ppm) 10.70 (s, 1H), 7.35-7.24 (m, 4H), 7.04-6.91 (m, 4H), 4.78 (d, *J* = 4.4 Hz, 1H), 4.07 (s, 2H), 4.01 (s, 1H), 3.81 (s, 3H), 3.29-3.27 (m, 1H), 3.06-2.99 (m, 2H), 2.88 (d, *J* = 11.2 Hz, 1H), 2.77-2.74 (m, 1H), 2.61-2.57 (m, 1H), 2.20-2.10 (m, 3H), 1.80-1.67 (m, 2H), 1.57-1.46 (m, 2H), 1.34-1.24 (m, 2H), 1.09-1.01 (m, 1H) |
| **C5** | 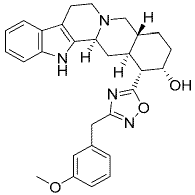 | ¹H-NMR (DMSO-*d₆*, 400 MHz): δ (ppm) 10.70 (s, 1H), 7.34 (d, *J* = 7.6 Hz, 1H), 7.28-7.23 (m, 2H), 7.01-6.91 (m, 4H), 6.84 (d, *J* = 7.6 Hz, 1H), 4.79 (s, 1H), 4.12 (s, 2H), 4.01 (s, 1H), 3.74 (s, 3H), 3.29-3.26 (m, 1H), 3.08-2.99 (m, 2H), 2.88 (d, *J* = 10.4 Hz, 1H), 2.77-2.74 (m, 1H), 2.61-2.57 (m, 1H), 2.19-2.11 (m, 3H), 1.80-1.77 (m, 1H), 1.69-1.64 (m, 1H), 1.56-1.47 (m, 2H), 1.35-1.24 (m, 2H), 1.09-1.01 (m, 1H) |
| **C6** | 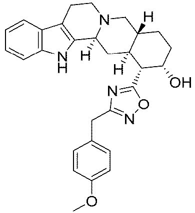 | ¹H-NMR (CDCl₃, 400 MHz): δ (ppm) 7.67 (s, 1H), 7.47 (d, *J* = 7.2 Hz, 1H), 7.34-7.29 (m, 3H), 7.17-7.08 (m, 2H), 6.93 (d, *J* = 8.4 Hz, 2H), 4.22 (s, 1H), 4.10 (s, 2H), 3.82 (s, 3H), 3.64 (s, 1H), 3.29 (d, *J* = 10.8 Hz, 1H), 3.14-3.09 (m, 1H), 3.01-2.99 (m, 2H), 2.93-2.90 (m, 1H), 2.75-2.71 (m, 1H), 2.67-2.60 (m, 1H), 2.30-2.45 (m, 1H), 2.14-2.08 (m, 2H), 1.75-1.65 (m, 4H), 1.52-1.40 (m, 2H) |
| **C7** | 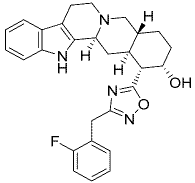 | ¹H-NMR (CDCl₃, 400 MHz): δ (ppm) 7.67 (s, 1H), 7.48 (d, *J* = 7.6 Hz, 1H), 7.41-7.31 (m, 3H), 7.20-7.08 (m, 4H), 4.24 (s, 1H), 4.22 (s, 2H), 3.62 (s, 1H), 3.30 (d, *J* = 11.2 Hz, 1H), 3.14-3.10 (m, 1H), 3.02-2.92 (m, 3H), 2.75-2.72 (m, 1H), 2.67-2.61 (m, 1H), 2.27-2.22 (m, 1H), 2.14-2.08 (m, 2H), 1.76-1.66 (m, 4H), 1.56-1.41 (m, 2H) |
| **C8** | 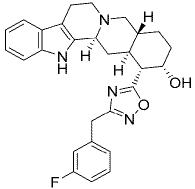 | ¹H-NMR (DMSO-*d₆*, 400 MHz): δ (ppm) 10.68 (s, 1H), 7.43-7.37 (m, 1H), 7.34 (d, *J* = 7.6 Hz, 1H), 7.25-7.23 (m, 3H), 7.12 (t, *J* = 8.2 Hz, 1H), 6.99 (t, *J* = 7.4 Hz, 1H), 6.93 (t, *J* = 7.2 Hz, 1H), 4.80 (d, *J* = 4.8 Hz, 1H), 4.19 (s, 2H), 4.01 (s, 1H), 3.29-3.27 (m, 1H), 3.09-2.99 (m, 2H), 2.89 (d, *J* = 9.2 Hz, 1H), 2.81-2.74 (m, 1H), 2.61-2.57 (m, 1H), 2.18-2.11 (m, 3H), 1.80-1.77 (m, 1H), 1.70-1.65 (m, 1H), 1.56-1.47 (m, 2H), 1.35-1.24 (m, 2H), 1.08-1.01 (m, 1H) |
| **C9** | 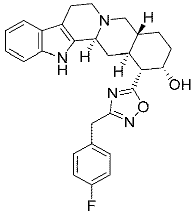 | ¹H-NMR (CDCl₃, 400 MHz): δ (ppm) 7.61 (s, 1H), 7.48 (d*, J* = 7.6 Hz, 1H), 7.40-7.37 (m, 2H), 7.32 (d, *J* = 7.6 Hz, 1H), 7.18-7.07 (m, 4H), 4.24 (s, 1H), 4.19-4.10 (m, 2H), 3.56 (s, 1H), 3.31 (d, *J* = 11.2 Hz, 1H), 3.14-2.93 (m, 4H), 2.76-2.61 (m, 2H), 2.31-2.22 (m, 1H), 2.15-2.03 (m, 2H), 1.75-1.66 (m, 4H), 1.53-1.42 (m, 2H) |
| **C10** | 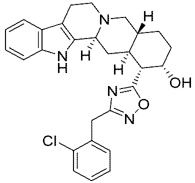 | ¹H-NMR (DMSO-*d₆*, 400 MHz): δ (ppm) 10.70 (s, 1H), 7.49-7.48 (m, 2H), 7.35 (m, 3H), 7.25 (d, *J* = 7.6 Hz, 1H), 6.99-6.91 (m, 2H), 4.80 (s, 1H), 4.26 (s, 2H), 4.01 (s, 1H), 3.30-3.27 (m, 1H), 3.09-3.01 (m, 2H), 2.89 (d, *J* = 9.6 Hz, 1H), 2.81-2.75 (m, 1H), 2.61-2.58 (m, 1H), 2.19-2.11 (m, 3H), 1.80-1.64 (m, 2H), 1.57-1.46 (m, 2H), 1.34-1.01 (m, 3H) |
| **C11** | 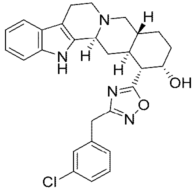 | ¹H-NMR (CDCl₃, 400 MHz): δ (ppm) 7.63 (s, 1H), 7.48 (d, *J* = 7.6 Hz, 1H), 7.39-7.31 (m, 5H), 7.18-7.08 (m, 2H), 4.25 (s, 1H), 4.15 (s, 2H), 3.47 (s, 1H), 3.33 (d, *J* = 10.8 Hz, 1H), 3.15-2.95 (m, 4H), 2.76-2.62 (m, 2H), 2.32-2.02 (m, 3H), 1.75-1.67 (m, 4H), 1.54-1.43 (m, 2H) |
| **C12** | 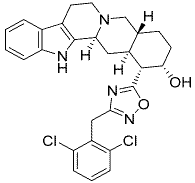 | ¹H-NMR (CDCl₃, 400 MHz): δ (ppm) 7.77 (s, 1H), 7.48-7.38 (m, 3H), 7.33-7.23 (m, 2H), 7.17-7.08 (m, 2H), 4.53 (s, 2H), 4.25 (s, 1H), 3.76 (s, 1H), 3.30 (d, *J* = 11.2 Hz, 1H), 3.14-3.10 (m, 1H), 3.02-2.99 (m, 2H), 2.92-2.90 (m, 1H), 2.76-2.72 (m, 1H), 2.67-2.62 (m, 1H), 2.30-2.25 (m, 1H), 2.11-2.08 (m, 2H), 1.79-1.65 (m, 4H), 1.52-1.46 (m, 2H) |
| **C13** | 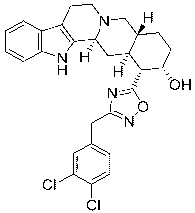 | ¹H-NMR (CDCl₃, 400 MHz): δ (ppm) 7.63 (s, 1H), 7.50-7.46 (m, 3H), 7.32 (d, *J* = 7.6 Hz, 1H), 7.26 (dd, *J* = 7.6 Hz, 1.0 Hz, 1H), 7.17-7.08 (m, 2H), 4.24 (s, 1H), 4.13 (s, 2H), 3.45 (s, 1H), 3.32 (d, *J* = 11.2 Hz, 1H), 3.14-3.10 (m, 1H), 3.05-2.95 (m, 3H), 2.77-2.61 (m, 2H), 2.32-2.07 (m, 3H), 1.76-1.66 (m, 4H), 1.54-1.43 (m, 2H) |
| **C14** | 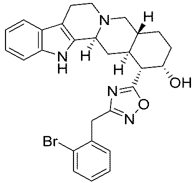 | ¹H-NMR (CDCl₃, 400 MHz): δ (ppm) 7.67-7.65 (m, 2H), 7.49-7.28 (m, 4H), 7.22 (t, *J* = 7.2 Hz, 1H), 7.17-7.08 (m, 2H), 4.34 (s, 2H), 4.25 (s, 1H), 3.64 (s, 1H), 3.29 (d, *J* = 10.8 Hz, 1H), 3.13-3.09 (m, 1H), 3.01-2.92 (m, 3H), 2.75-2.71 (m, 1H), 2.67-2.60 (m, 1H), 2.29-2.24 (m, 1H), 2.14-2.08 (m, 2H), 1.76-1.62 (m, 5H), 1.51-1.41 (m, 1H) |
| **C15** | 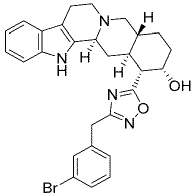 | ¹H-NMR (CDCl₃, 400 MHz): δ (ppm) 7.72 (s, 1H), 7.55 (s, 1H), 7.48-7.46 (m, 2H), 7.36-7.25 (m, 3H), 7.17-7.08 (m, 2H), 4.22 (s, 1H), 4.14 (s, 2H), 3.49 (s, 1H), 3.31 (d, *J* = 10.4 Hz, 1H), 3.14-3.10 (m, 1H), 3.02-2.99 (m, 2H), 2.93-2.90 (m, 1H), 2.75-2.72 (m, 1H), 2.68-2.62 (m, 1H), 2.30-2.25 (m, 1H), 2.14-2.08 (m, 2H), 1.75-1.65 (m, 4H), 1.52-1.40 (m, 2H) |
| **C16** | 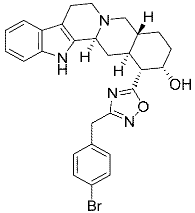 | ¹H-NMR (DMSO-*d₆*, 400 MHz): δ (ppm) 10.68 (s, 1H), 7.56-7.54 (m, 2H), 7.36-7.35 (m, 3H), 7.28-7.24 (m, 1H), 6.99-6.93 (m, 2H), 4.78 (s, 1H), 4.14 (s, 2H), 4.00 (s, 1H), 3.29-3.26 (m, 1H), 3.07-3.02 (m, 2H), 2.88 (d, *J* = 9.6 Hz, 1H), 2.77-2.74 (m, 1H), 2.61-2.57 (m, 1H), 2.17-2.15 (m, 3H), 1.80-1.51 (m, 4H), 1.34-1.24 (m, 2H), 1.06-1.03 (m, 1H) |
| **C20** | 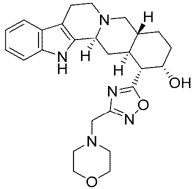 | ¹H-NMR (CDCl₃, 400 MHz): δ (ppm) 8.09 (s, 1H), 7.47 (d, J = 7.2 Hz, 1H), 7.32 (d, J = 8.0 Hz, 1H), 7.17-7.07 (m, 2H), 4.29 (s, 1H), 3.86- 3.81 (m, 6H), 3.42-3.40 (m, 1H), 3.19-3.18 (m, 1H), 3.09-2.99 (m, 3H), 2.79-2.69 (m, 6H), 2.33 (t, J = 7.4 Hz, 2H), 2.22-2.10 (m, 2H), 1.88-1.48 (m, 6H) |
| **C21** | 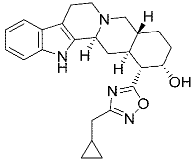 | ¹H-NMR (CDCl₃, 400 MHz): δ (ppm) 7.73 (s, 1H), 7.48 (d, J = 7.2 Hz, 1H), 7.31 (d, J = 7.6 Hz, 1H), 7.17-7.10 (m, 2H), 4.27 (s, 1H), 3.92 (s, 1H), 3.31 (d, J = 10.8 Hz, 1H), 3.13-3.10 (m, 1H), 3.03-2.94 (m, 3H), 2.76-2.63 (m, 4H), 2.31-2.11 (m, 3H), 1.76-1.70 (m, 4H), 1.52-1.46 (m, 2H), 1.23-1.20 (m, 1H), 0.67 (d, J = 8.0 Hz, 2H), 0.35 (s, 2H) |
| **C22** | 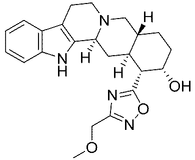 | ¹H-NMR (CDCl₃, 400 MHz): δ (ppm) 7.84 (s, 1H), 7.48 (d, J = 7.6 Hz, 1H), 7.31 (d, J = 8.0 Hz, 1H), 7.16-7.07 (m, 2H), 4.68 (s, 2H), 4.26 (s, 1H), 3.59 (s, 3H), 3.30 (d, J = 10.8 Hz, 1H), 3.14-3.10 (m, 1H), 3.06-2.97 (m, 3H), 2.76-2.62 (m, 2H), 2.30-2.10 (m, 3H), 1.91-1.85 (m, 1H), 1.77-1.69 (m, 4H), 1.53-1.42 (m, 2H) |
| **C23** | 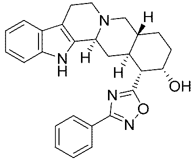 | ¹H-NMR (CDCl₃, 400 MHz): δ (ppm) 8.16 (d, J = 6.8 Hz, 2H), 7.77 (s, 1H), 7.57-7.55 (m, 3H), 7.48 (d, J = 7.2 Hz, 1H), 7.29-7.27 (m, 1H), 7.15-7.07 (m, 2H), 4.31 (s, 1H), 3.88 (s, 1H), 3.32 (d, J = 10.4 Hz, 1H), 3.14- 3.10 (m, 1H), 3.05-3.02 (m, 3H), 2.76-2.63 (m, 2H), 2.33-2.14 (m, 3H), 1.76-1.67 (m, 4H), 1.57-1.48 (m, 2H) |
| **C24** | 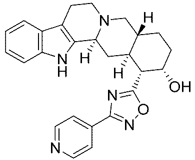 | ¹H-NMR (CDCl₃ + CD₃OD, 400 MHz): δ (ppm) 8.76 (d, J = 5.6 Hz, 2H), 8.70 (s, 1H), 7.94 (d, J = 6.0 Hz, 2H), 7.49-7.47 (m, 1H), 7.22- 7.20 (m, 1H), 7.11-7.08 (m, 2H), 4.30 (s, 1H), 3.71 (s, 1H), 3.46 (d, J = 10.8 Hz, 1H), 3.20-3.07 (m, 3H), 2.80-2.71 (m, 2H), 2.37-2.14 (m, 4H), 1.73-1.56 (m, 6H) |
| **C25** | 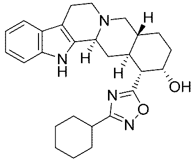 | ¹H-NMR (CDCl₃, 400 MHz): δ (ppm) 7.75 (s, 1H), 7.48 (d, J = 7.2 Hz, 1H), 7.31 (d, J = 7.6 Hz, 1H), 7.16-7.08 (m, 2H), 4.24 (s, 1H), 4.02 (s, 1H), 3.30 (d, J = 10.4 Hz, 1H), 3.12-2.86 (m, 5H), 2.76-2.62 (m, 2H), 2.28 (t, J = 9.6 Hz, 1H), 2.13-2.12 (m, 4H), 1.91-1.61 (m, 10H), 1.51-1.41 (m, 4H) |
| **C26** | 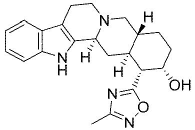 | ¹H-NMR (CDCl₃, 400 MHz): δ (ppm) 7.77 (s, 1H), 7.48 (d, J = 7.2 Hz, 1H), 7.33-7.31 (m, 1H), 7.17-7.08 (m, 2H), 4.29 (s, 1H), 3.73 (s, 1H), 3.35 (d, J = 10.4 Hz, 1H), 3.17-2.93 (m, 4H), 2.78-2.68 (m, 2H), 2.51 (s, 3H), 2.33-2.29 (m, 1H), 2.13-2.11 (m, 2H), 1.76-1.67 (m, 4H), 1.54-1.45 (m, 2H) |
| **C28** | 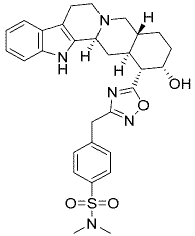 | ¹H-NMR (CDCl₃, 400 MHz): δ (ppm) 7.78 (d, J = 8.4 Hz, 2H), 7.64 (s, 1H), 7.56 (d, J = 8.4 Hz, 2H), 7.45 (d, J = 7.6 Hz, 1H), 7.30 (d, J = 8.0 Hz, 1H), 7.16-7.11 (m, 1H), 7.11-7.05 (m, 1H), 4.30-4.17 (m, 3H), 3.50 (s, 1H), 3.31 (d, J = 11.2 Hz, 1H), 3.14-3.07 (m, 1H), 3.04-2.90 (m, 3H), 2.76-2.68 (m, 7H), 2.68-2.60 (m, 1H), 2.31-2.25 (m, 1H), 2.16-2.03 (m, 2H), 1.76-1.64 (m, 4H), 1.52-1.41 (m, 2H) |
| **C29** | 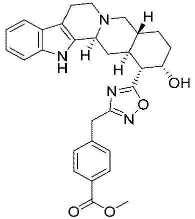 | ¹H-NMR (CDCl₃, 500 MHz): δ (ppm) 8.05 (d, J = 8.5 Hz, 2H), 7.57 (s, 1H), 7.48-7.43 (m, 3H), 7.28 (d, J = 7.5 Hz, 1H), 7.15-7.10 (m, 1H), 7.10-7.05 (m, 1H), 4.24-4.15 (m, 3H), 3.91 (s, 3H), 3.28 (d, J = 11.0 Hz, 1H), 3.09 (dd, J = 11.3, 5.8 Hz, 1H), 3.02-2.94 (m, 2H), 2.92 (dd, J = 11.5, 2.0 Hz, 1H), 2.71 (dd, J = 15.3, 3.8 Hz, 1H), 2.62 (td, J = 11.3, 4.3 Hz, 1H), 2.26 (t, J = 10.5 Hz, 1H), 2.15-2.04 (m, 2H), 1.73-1.61 (m, 5H), 1.51-1.39 (m, 2H) |
| **C31** | 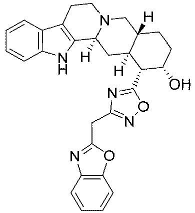 | ¹H-NMR (CDCl₃, 400 MHz): δ (ppm) 8.27 (s, 1H), 7.70 (d, *J* = 8.4 Hz, 1H), 7.55 (d, *J* = 6.8 Hz, 1H), 7.45 (d, *J* = 7.6 Hz, 1H), 7.40-7.31 (m, 2H), 7.28 (d, *J* = 8.0 Hz, 1H), 7.12 (t, *J* = 7.4 Hz, 1H), 7.06 (t, *J* = 7.2 Hz, 1H), 4.59-4.44 (m, 2H), 4.24 (s, 1H), 3.28 (d, *J* = 11.6 Hz, 1H), 3.12-3.04 (m, 1H), 3.04-2.93 (m, 3H), 2.71 (d, *J* = 15.6 Hz, 1H), 2.61 (td, *J* = 11.2, 4.4 Hz, 1H), 2.29-2.03 (m, 3H), 1.90-1.79 (m, 2H), 1.72-1.60 (m, 3H), 1.51-1.36 (m, 2H) |
| **C32** | 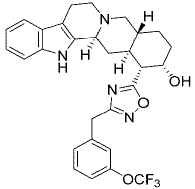 | ¹H-NMR (CDCl₃, 400 MHz): δ (ppm) 7.56 (s, 1H), 7.48-7.37 (m, 2H), 7.35-7.25 (m, 3H), 7.21-7.03 (m, 3H), 4.21 (s, 1H), 4.17 (s, 2H), 3.51 (s, 1H), 3.27 (d, J = 11.2 Hz, 1H), 3.13-3.04 (m, 1H), 3.03-2.90 (m, 3H), 2.71 (d, J = 14.8 Hz, 1H), 2.61 (td, J = 11.2, 4.2 Hz, 1H), 2.26 (t, J = 10.4 Hz, 1H), 2.19-2.04 (m, 2H), 1.74-1.60 (m, 4H), 1.54-1.38 (m, 2H) |
| **C33** | 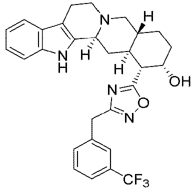 | ¹H-NMR (CDCl₃, 400 MHz): δ (ppm) 7.65-7.54 (m, 4H), 7.50 (d, J = 8.0 Hz, 1H), 7.44 (d, J = 7.6 Hz, 1H), 7.26 (d, J = 8.0 Hz, 1H), 7.12 (t, J = 7.0 Hz, 1H), 7.07 (t, J = 7.0 Hz, 1H), 4.23-4.14 (m, 3H), 3.47 (s, 1H), 3.26 (d, J = 10.8 Hz, 1H), 3.12-2.87 (m, 4H), 2.70 (dd, J = 15.2, 4.0 Hz, 1H), 2.60 (td, J = 11.2, 4.4 Hz, 1H), 2.24 (t, J = 10.4 Hz, 1H), 2.18-2.02 (m, 2H), 1.73-1.58 (m, 4H), 1.51-1.37 (m, 2H) |
| **C34** | 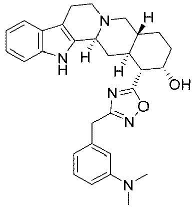 | ¹H-NMR (CDCl₃, 400 MHz): δ (ppm) 7.59 (s, 1H), 7.45 (d, J = 7.6 Hz, 1H), 7.29 (d, J = 7.6 Hz, 1H), 7.23 (d, J = 7.6 Hz, 1H), 7.13 (t, J = 7.4 Hz, 1H), 7.07 (t, J = 7.4 Hz, 1H), 6.80-6.59 (m, 3H), 4.22 (s, 1H), 4.09 (s, 2H), 3.61 (s, 1H), 3.27 (d, J = 11.6 Hz, 1H), 3.13-3.05 (m, 1H), 3.03-2.88 (m, 9H), 2.75-2.57 (m, 2H), 2.30-2.03 (m, 3H), 1.74-1.61 (m, 4H), 1.52-1.36 (m, 2H) |
| **C35** | 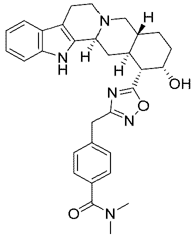 | ¹H-NMR (CDCl₃, 500 MHz): δ (ppm) 7.69 (s, 1H), 7.45 (d, J = 7.5 Hz, 1H), 7.43-7.39 (m, 4H), 7.27 (d, J = 7.5 Hz, 1H), 7.14-7.09 (m, 1H), 7.09-7.04 (m, 1H), 4.20 (s, 1H), 4.19-4.11 (m, 2H), 3.65 (s, 1H), 3.28 (d, J = 11.0 Hz, 1H), 3.15-3.03 (m, 4H), 3.03-2.93 (m, 5H), 2.91 (dd, J = 11.5, 2.0 Hz, 1H), 2.71 (dd, J = 15.3, 4.7 Hz, 1H), 2.61 (td, J = 11.3, 4.5 Hz, 1H), 2.25 (t, J = 10.8 Hz, 1H), 2.15-2.04 (m, 2H), 1.75-1.60 (m, 4H), 1.51-1.39 (m, 2H) |

### Example 2

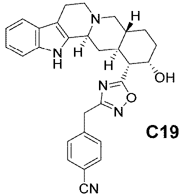

(1) At room temperature, 4-iodobenzeneacetonitrile (2.43 g, 10 mmol) was dissolved in ethanol (20 mL), and water (8 mL), sodium carbonate (848 mg, 8 mmol), and hydroxylamine hydrochloride (695 mg, 10 mmol) were added in sequence. After the addition, the mixture was stirred at room temperature for 24 hours, and most of the solvent was removed by concentration under reduced pressure. The residue was dissolved in ethyl acetate (80 mL), washed with saturated brine (20 mL × 2), and the organic phase was dried over anhydrous sodium sulfate and concentrated under reduced pressure. The product was directly used for condensation reaction without purification.
(2) At room temperature, M2 (340 mg, 1 mmol) was dissolved in DMF (5 mL), triethylamine (304 mg, 3 mmol) was added, and COMU (428 mg, 1 mmol) was slowly added after stirring for 5 minutes. After the addition was completed, the mixture was stirred at room temperature for 20 minutes, and the crude product (552 mg, 2 mmol) in operation (1) was added. After stirring at room temperature for 24 hours, the reaction solution was concentrated under reduced pressure to remove the solvent. The residue was dissolved in dichloromethane/methanol (100 mL/10 mL), washed with saturated brine (20 mL×3), and the organic phase was dried over anhydrous sodium sulfate, concentrated under reduced pressure, and purified by column chromatography (dichloromethane/methanol = 30:1) to obtain a brown foamy solid M3.
(3) The condensation product M3 in operation (2) was dissolved in 1,4-dioxane (5 mL), and toluene (10 mL) was added. The mixture was heated to 100 °C under a nitrogen atmosphere. The reaction was stopped after 24 hours. The solvent was removed by concentration under reduced pressure. The residue was dissolved in dichloromethane/methanol (100 mL/10 mL), washed with saturated brine (20 mL×3), and the organic phase was dried over anhydrous sodium sulfate, concentrated under reduced pressure, and purified by column chromatography (dichloromethane/methanol=30:1) to obtain a light brown foamy solid M4 (69 mg, 0.12 mmol). The molar yield of operations (2) and (3) was 12%.
(4) M4 (58 mg, 0.1 mmol) was dissolved in DMF (5 mL), and Pd(dba)₂ (5.7 mg, 0.01 mmol), dppf (5.5 mg, 0.01 mmol), and Zn(CN)₂ (11.7 mg, 0.1 mmol) were added. The mixture was heated to 80 °C under a nitrogen atmosphere. The reaction was stopped after 6 hours. The solvent was removed by concentration under reduced pressure. The residue was dissolved in dichloromethane/methanol (100 mL/10 mL), washed with saturated brine (20 mL×3), and the organic phase was dried over anhydrous sodium sulfate, concentrated under reduced pressure, and purified by column chromatography (dichloromethane/methanol = 30:1) to obtain light brown foamy solid C19 (33 mg, 0.07 mmol) with a molar yield of 70%.

The other compounds in the following table were synthesized using the same method as in Example 2 with different cyano substrates:

| Compound | Structure | ¹H-NMR |
|---|---|---|
| **C17** | 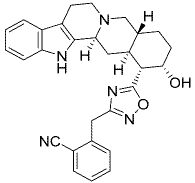 | ¹H-NMR (CDCl₃, 400 MHz): δ (ppm) 8.48 (s, 1H), 7.77 (d, *J* = 7.6 Hz, 1H), 7.83 (t, *J* = 7.2 Hz, 1H), 7.56-7.46 (m, 3H), 7.34 (d, *J* = 8.0 Hz, 1H), 7.15-7.05 (m, 2H), 4.46-3.36 (m, 2H), 4.27 (s, 1H), 3.33 (d, *J* = 10.4 Hz, 1H), 3.16-3.00 (m, 4H), 2.76-2.62 (m, 2H), 2.31-2.07 (m, 3H), 1.89-1.54 (m, 5H), 1.42-1.28 (m, 2H) |
| **C18** | 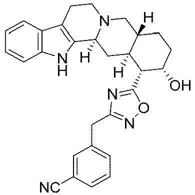 | ¹H-NMR (CDCl₃, 400 MHz): δ (ppm) 7.70-7.63 (m, 4H), 7.54-7.47 (m, 2H), 7.31 (d, *J* = 8.0 Hz, 1H), 7.17-7.08 (m, 2H), 4.26-4.17 (m, 3H), 3.44 (s, 1H), 3.32 (d, *J* = 10.8 Hz, 1H), 3.14-3.10 (m, 1H), 3.03-2.95 (m, 2H), 2.76-2.61 (m, 2H), 2.32-2.26 (m, 1H), 2.16-2.08 (m, 2H), 1.77-1.70 (m, 5H), 1.53-1.43 (m, 2H) |
| **C19** | 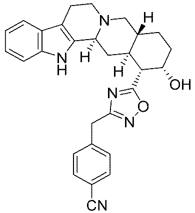 | ¹H-NMR (DMSO-*d*₆, 400 MHz): δ(ppm) 10.69 (s, 1H), 7.83 (d, *J* = 7.6 Hz, 2H), 7.60 (d, *J* = 7.6 Hz, 2H), 7.33 (d, *J* = 7.6 Hz, 1H), 7.24 (d, *J* = 8.0 Hz, 1H), 7.02-6.95 (m, 1H), 6.95-6.88 (m, 1H), 4.80 (d, *J* = 4.8 Hz, 1H), 4.28 (s, 2H), 3.99 (s, 1H), 3.29-3.22 (m, 1H), 3.07 (d, *J* = 12.0 Hz, 1H), 3.04-2.97 (m, 1H), 2.88 (d, *J* = 10.8 Hz, 1H), 2.81-2.70 (m, 1H), 2.64-2.51 (m, 2H), 2.22-2.06 (m, 3H), 1.82-1.73 (m, 1H), 1.72-1.62 (m, 1H), 1.57-1.44 (m, 2H), 1.39-1.30 (m, 1H), 1.09-0.98 (m, 1H) |

### Example 3

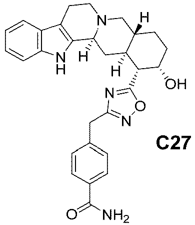

Compound C19 (14 mg, 0.03 mmol) was dissolved in EtOH (0.15 mL), and DMSO (0.075 mL) was added under ice bath, followed by 4 M NaOH (0.0073 mL) and 30% H₂O₂ (0.01 mL). After reacting under ice bath for 30 minutes, the mixture was brought to room temperature. The reaction was stopped after 5 hours. 10% Na₂S₂O₃ (0.15 mL) was added to the reaction system under ice bath, followed by H₂O (2 mL). A white solid precipitated, and the mixture was filtered under reduced pressure. The filter cake was washed with water and dried to obtain white solid C27 (10 mg, 0.02 mmol) with a molar yield of 67%.

| Compound | structure | ¹H-NMR |
|---|---|---|
| **C27** | 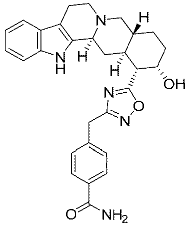 | ¹H-NMR (DMSO-*d*₆, 400 MHz): δ(ppm) 10.69 (s, 1H), 7.95 (s, 1H), 7.85 (d, *J* = 8.0 Hz, 2H), 7.45 (d, *J* = 8.0 Hz, 2H), 7.37-7.30 (m, 2H), 7.24 (d, *J* = 8.0 Hz, 1H), 7.01-6.96 (m, 1H), 6.95-6.89 (m, 1H), 4.79 (d, *J* = 4.8 Hz, 1H), 4.20 (s, 2H), 4.03-3.96 (m, 1H), 3.27 (d, *J* = 10.8 Hz, 1H), 3.06 (dd, *J* = 11.6, 2.4 Hz, 1H), 3.03-2.96 (m, 1H), 2.88 (d, *J* = 10.8 Hz, 1H), 2.82-2.71 (m, 1H), 2.63-2.55 (m, 1H), 2.54-2.50 (m, 1H), 2.22-2.06 (m, 3H), 1.81-1.73 (m, 1H), 1.70-1.61 (m, 1H), 1.56-1.43 (m, 2H), 1.38-1.29 (m, 1H), 1.04 (q, *J* = 11.6 Hz, 1H) |

### Example 4

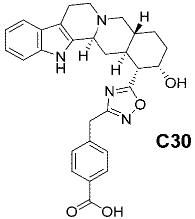

Compound C29 (50 mg, 0.1 mmol) was placed in methanol/tetrahydrofuran/water (2.1 mL/0.3 mL/0.6 mL) under ice bath, lithium hydroxide monohydrate (42 mg, 1 mmol) was added, and the mixture was stirred under ice bath for half an hour and then moved to room temperature. The reaction was monitored by LC-MS until the starting material disappeared, and most of the solvent was removed by concentration under reduced pressure. Water (5 mL) was added to the residue, and the pH was adjusted to 6 with 5% hydrochloric acid under ice bath. The mixture was allowed to stand and filtered. The filter cake was washed with water (2 mL), and the filter cake was collected and dried to obtain yellow powder C30 (25 mg, 0.05 mmol) with a molar yield of 50%.

| compound | structure | ¹H-NMR |
|---|---|---|
| **C30** | 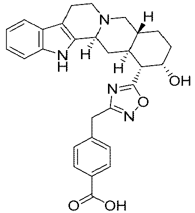 | ¹H-NMR (DMSO-*d*₆, 500 MHz): δ(ppm) 12.77 (s, 1H), 10.71 (s, 1H), 7.91 (d, J = 8.0 Hz, 2H), 7.49 (d, J = 8.0 Hz, 2H), 7.33 (d, J = 7.5 Hz, 1H), 7.24 (d, J = 8.0 Hz, 1H), 6.99 (t, J = 7.5 Hz, 1H), 6.91 (t, J = 7.5 Hz, 1H), 4.79 (d, J = 5.0 Hz, 1H), 4.27-4.17 (m, 2H), 4.03-3.95 (m, 1H), 3.13-2.99 (m, 2H), 2.96-2.88 (m, 1H), 2.82-2.73 (m, 1H), 2.65-2.52 (m, 2H), 2.32-2.09 (m, 3H), 1.79-1.72 (m, 1H), 1.69-1.60 (m, 1H), 1.56-1.42 (m, 2H), 1.39-1.29 (m, 1H), 1.18-1.02 (m, 2H) |

### Example 5 α2A-AR antagonist assay

### 1. Purpose of the experiment

The Purpose is to investigate antagonistic activity of yohimbine derivatives on α2A-AR.

### 2. Experimental Principle

By establishing a cell line co-transfected with the target receptor and Ga16, the receptor can be activated to cause the activation of Gα16 protein, which in turn activates phospholipase C (PLC) to produce IP3 and DAG. IP3 can bind to the IP3 receptors on the endoplasmic reticulum and mitochondria in the cell, thereby causing the release of intracellular calcium. Therefore, measuring the changes in intracellular calcium can be used as a method to detect the activation state of the target receptor. Fluo-4/AM is a calcium fluorescent probe indicator used to measure calcium ions. As a non-polar lipid-soluble compound, after entering the cell, the AM group dissociates under the action of cell lipases and releases Fluo-4; since Fluo-4 is a polar molecule that is not easy to pass through the lipid bilayer membrane, it can keep in the cell for a long time. Finally, the level of Ga protein activation can be reflected by measuring the excited fluorescence intensity. If the screened compound can excite the target receptor, the calcium flow reaction can be greatly increased; conversely, if the screened compound can antagonize the target receptor, the calcium flow reaction can be greatly reduced.

### 3. Experimental samples

Before the test, the test compound was dissolved in DMSO to prepare a stock solution, which was diluted with culture medium to the required concentration when used.

### 4. Experimental Methods

Cells stably expressing α2A-AR/Ga16 were seeded in 96-well plates and cultured overnight; the culture medium in the wells with cells was aspirated, and freshly prepared dye (40 µL/well) was added, and incubated in a 37 °C incubator for 40 minutes; the drug to be tested was diluted and mixed with calcium buffer; the dye was aspirated and discarded, and the well was washed with freshly prepared calcium buffer, and then replaced with 50 µL of calcium buffer dissolved with the drug to be tested; the FlexStation II instrument was used for detection, and 25 µL of calcium buffer dissolved with a known agonist UK14304 was automatically added by the instrument starting from the 15th second, and the fluorescence value at 525 nm was finally read.

### 5. Experimental results (taking the 35 compounds in Table 1 as an example, but not limited to these compounds)

**Table 1 Test results of α2A-AR antagonistic activity of compounds**

| Compound No. | α2A-AR (IC₅₀/µM) |
|---|---|
| **M1** (yohimbine hydrochloride) | ** |
| **C1** | **** |
| **C2** | ** |
| **C3** | *** |
| **C4** | * |
| **C5** | *** |
| **C6** | **** |
| **C7** | * |
| **C8** | ** |
| **C9** | * |
| **C10** | * |
| **C11** | * |
| **C12** | *** |
| **C13** | * |
| **C14** | **** |
| **C15** | **** |
| **C16** | ***** |
| **C17** | *** |
| **C18** | **** |
| **C19** | ** |
| **C20** | *** |
| **C21** | * |
| **C22** | *** |
| **C23** | ** |
| **C24** | * |
| **C25** | ** |
| **C26** | *** |
| **C27** | **** |
| **C28** | **** |
| **C29** | *** |
| **C30** | ** |
| **C31** | ** |
| **C32** | **** |
| **C33** | *** |
| **C34** | *** |
| **C35** | *** |

| | |
|---|---|
| Note: IC₅₀ is the evaluation of the sample drug's antagonistic activity on α2A-AR. * represents 0.5µM ≤ IC₅₀ < 2µM; ** represents 0.3µM ≤ IC₅₀ <0.5µM, *** represents 0.1 µM ≤ IC₅₀ <0.3 µM; **** represents IC₅₀ <0.1 µM. | |

### 6. Results and Discussion

These compounds can compete with the α2A-AR agonist UK14304 in cells expressing α2A-AR and antagonize the agonistic effect of UK14304 on α2A-AR. IC₅₀ is shown in Table 1. The results show that these compounds are antagonists of α2A-AR.

### Example 6 Yohimbine derivatives distribution test in mouse tissue

### 1. Purpose of the experiment

The purpose is to investigate the tissue distribution of yohimbine derivatives in mice.

### 2. Experimental Design

**Table 2 Dosage regimen for mouse tissue distribution test**

| Group | Anima No. | Administered compound | Administration route | Administration dosage (mg/kg) | Administration volume (ml/kg) |
|---|---|---|---|---|---|
| 1 | 1-3 | **M1** (yohimbine hydrochloride) | gavage | 10 | 10 |
| 2 | 4-6 | **C1** | gavage | 10 | 10 |
| 3 | 7-9 | **C6** | gavage | 10 | 10 |
| 4 | 10-12 | **C13** | gavage | 10 | 10 |
| 5 | 13-15 | **C19** | gavage | 10 | 10 |

| | | | | | |
|---|---|---|---|---|---|
| Note: All the administered compounds were in the form of hydrochloride, prepared with deionized water; all the experimental animals were ICR males | | | | | |

Mice were fasted for more than 12 h and had free access to water.

**Table 3 Experimental Scheme for tissue distribution of Compound C27 in mice**

| Group | Animal No. | Administered compound | Administration route | Time/h | Administration dosage | Administration volume |
|---|---|---|---|---|---|---|
| | | | | | (mg/kg) | (ml/kg) |
| 1 | 16 - 18 | **C27** | gavage | 0.25 | 10 | 10 |
| 2 | 19 - 21 | | | 2 | | |
| 3 | 22 - 24 | | | 8 | | |

| | | | | | | |
|---|---|---|---|---|---|---|
| Note: The solvent is 5% DMSO + 5% solutol + 90% saline; the experimental animals are all ICR males | | | | | | |

Mice were fasted for more than 12 h and had free access to water.

### 3. Sample collection and determination

(1) Tissue distribution test in mice: The animals in each group were anesthetized and killed 2 h after administration. The brain, pancreas, heart, liver, and kidney tissues were collected by dissection, washed with physiological saline, and stored in a -20 °C refrigerator for testing. At the same time, 0.3 mL of whole blood was collected and placed in an EDTA-K2 anticoagulant tube. The blood was separated by centrifugation at 11,000 rpm for 5 min and stored in a -20 °C refrigerator for testing.

Blank plasma and tissues were collected from another 3 animals.

The concentration of unchanged drug in plasma and tissues was determined.

(2) Tissue distribution test of compound C27 in mice: At corresponding time points (0.25, 2 and 8 h) after gavage administration, mice were bled to death through the abdominal aorta. Brain, heart, liver, lung, pancreas and kidney tissues were immediately collected by dissection, and part of the plasma was collected. The operation was carried out in an ice water bath.

Centrifugation conditions: 11000 rpm for 5 min to separate plasma (about 200 µL).

After collection, tissues and plasma were stored below -60 °C.

### 4. Experimental results and discussion

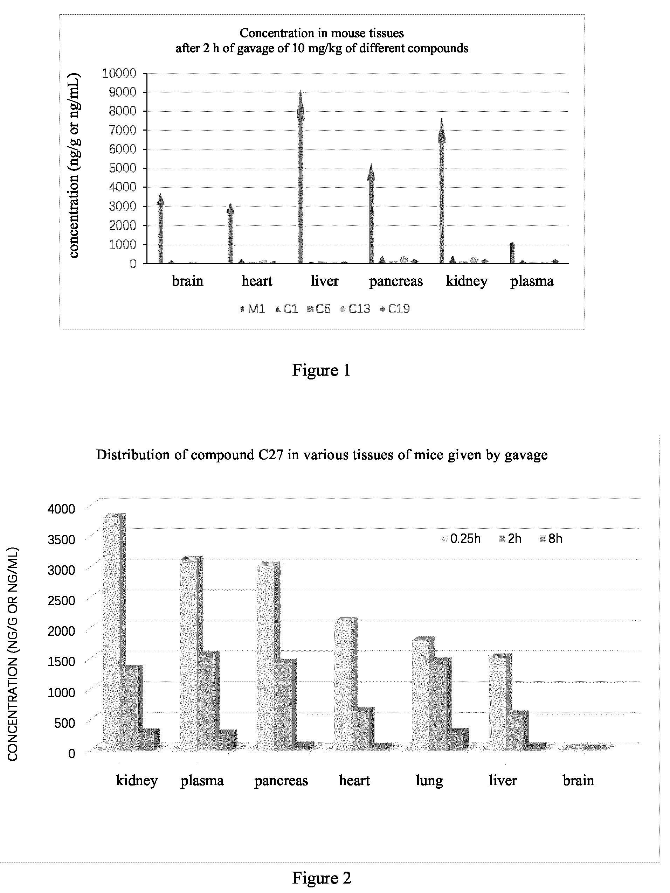

Figure 1 shows the tissue concentration (ng/g or ng/mL) of different compounds in mice 2 hours after gavage administration of 10 mg/kg of different compounds. (The four compounds in Figure 1 are used as examples, but are not limited to these compounds)

Figure 2 shows the tissue concentrations (ng/g or ng/mL) of compound C27 in mice 0.25, 2 and 8 hours after gavage administration of 10 mg/kg of compound C27.

Calculated by the relative ratio of tissue distribution concentration to blood concentration, compounds C1, C6, C13, C19, and C27 all showed lower brain tissue distribution than yohimbine, among which the brain tissue distribution of compound C19 was about forty times lower than that of yohimbine, and compound C19 also had good pancreatic distribution; the plasma exposure of compound C27 was equivalent to that of yohimbine, but its brain tissue distribution was about two hundred and seventy times lower than that of yohimbine, and it had pancreatic distribution characteristics comparable to those of compound C19.

### Example 7 Experimental test of the therapeutic effect of yohimbine derivatives on lowering blood sugar in diabetic mice

### 1. Purpose of the experiment

To test whether the yohimbine derivative C19 can improve blood sugar metabolism in type 2 diabetes

### 2. Experimental content

A high-sugar and high-fat diet-induced type 2 diabetes mouse model was used in this experiment, and the following tests were mainly conducted:
1) Effect of single intraperitoneal administration on basal blood glucose, glucose tolerance and insulin sensitivity in diabetic mice.
2) Effect of single gavage administration on glucose tolerance and insulin sensitivity in diabetic mice.
3) Effect of continuous chronic intraperitoneal administration on basal blood glucose in diabetic mice.

### 3. Experimental Methods

C57BL\6J mice were fed with a high-sugar and high-fat diet for 3 consecutive months. The test experiment was conducted after the average body weight of the mice stabilized at more than 50g and the average basal blood sugar value stabilized at more than 12mmol/L.

Blood glucose level was mainly measured by measuring tail vein blood using a blood glucose meter. For the test of basal blood glucose effect, the mice were on a normal diet. The basal blood glucose value before administration was first measured, and then a single intraperitoneal injection or a single gavage of the yohimbine derivative C19 (10 mg/kg mouse body weight) was given, and the venous blood glucose level was measured 15, 30, 60, 90, and 120 minutes after administration. The mouse glucose tolerance test was performed after the mouse has eaten for 16 hours overnight. 30 minutes after a single intraperitoneal injection or a single gavage of the yohimbine derivative C19 (10 mg/kg mouse body weight), glucose (1.5 g/kg) was injected intraperitoneally, and the venous blood glucose level was measured 15, 30, 60, 90, and 120 minutes later. Mouse insulin sensitivity was tested after the mouse has eaten for 6 hours. 30 minutes after a single intraperitoneal injection or single gavage of yohimbine derivative C19 (10 mg/kg mouse body weight), insulin (1 UI/kg) was injected intraperitoneally, and the venous blood glucose level was measured 15, 30, 60, 90, and 120 minutes later. For the effect of chronic administration on blood glucose levels, diabetic mice were intraperitoneally injected with yohimbine derivative C19 (10 mg/kg) every day for 12 consecutive days, and the basal blood glucose level of mice under free diet was measured every day before drug injection. The control group was injected with normal saline or gavage.

### 4. Experimental results

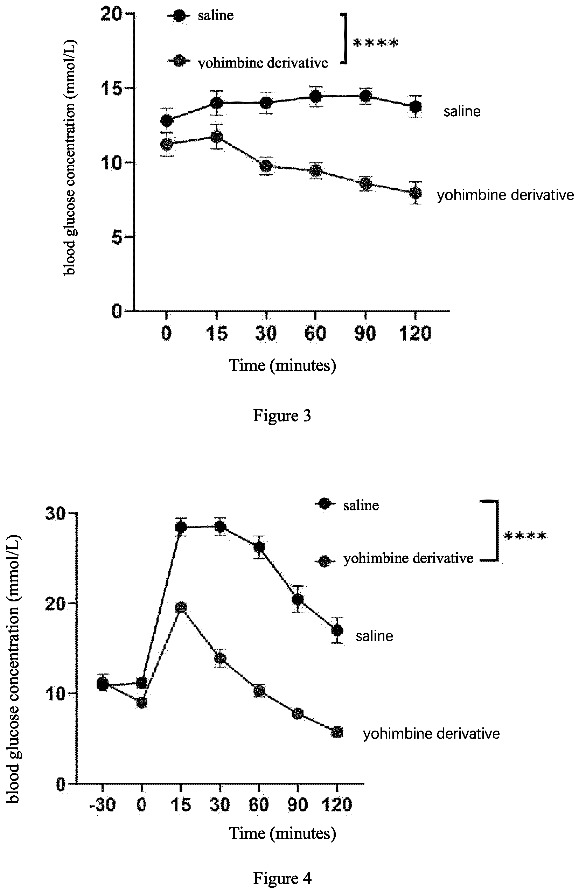

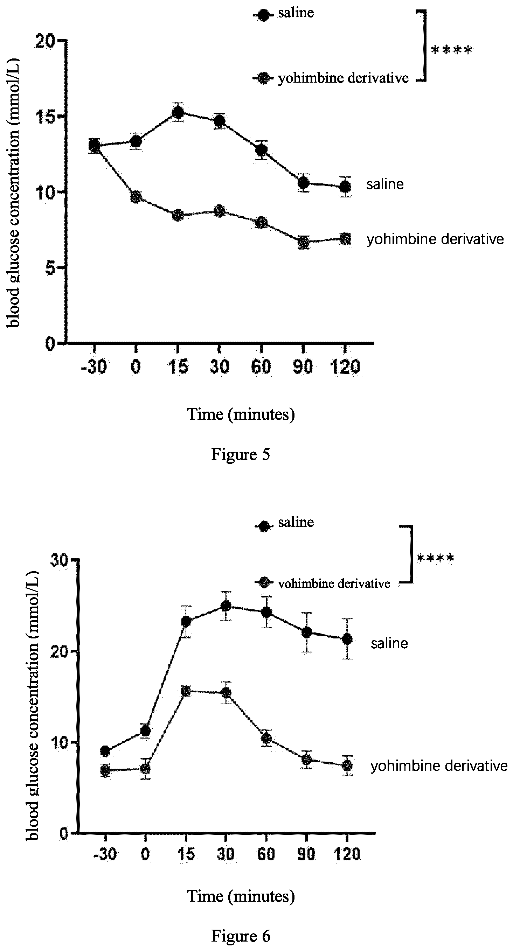

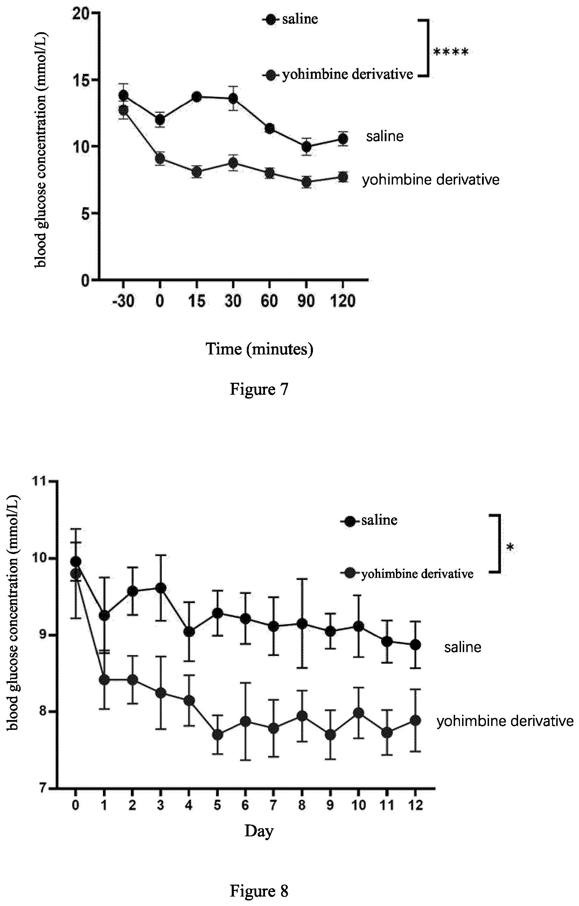

The experimental results are shown in Figures 3-8. The results of Figure 3 show that a single intraperitoneal injection of Yohimbine derivatives (10 mg/kg) significantly reduces the basal blood glucose level of type 2 diabetic mice. The results of Figure 4 show that a single intraperitoneal injection of Yohimbine derivatives (10 mg/kg) significantly improves the glucose tolerance of type 2 diabetic mice. The results of Figure 5 show that a single intraperitoneal injection of Yohimbine derivatives (10 mg/kg) significantly improves the insulin sensitivity of type 2 diabetic mice. The results of Figure 6 show that a single gavage administration of Yohimbine derivatives (10 mg/kg) significantly improves the glucose tolerance of type 2 diabetic mice. The results of Figure 7 show that a single gavage administration of Yohimbine derivatives (10 mg/kg) significantly improves the insulin sensitivity of type 2 diabetic mice. The results of Figure 8 show that chronic injection of Yohimbine derivatives (10 mg/kg, once a day) significantly reduces the basal blood glucose level of type 2 diabetic mice.

### 5 Results and discussion

This experiment shows that a single injection or gavage administration of the yohimbine derivative C19 of the present invention can significantly reduce the high blood sugar level of diabetic mice, improve glucose tolerance, and increase insulin sensitivity. Chronic administration can stably maintain the basal blood sugar level of diabetic mice at a state closer to the normal level. Therefore, the yohimbine derivative C19 of the present invention has a significant effect of improving diabetes blood sugar metabolism.

By structurally modifying yohimbine, a new derivative with enhanced α2A adrenaline receptor antagonist activity, pancreatic targeting and reduced brain tissue distribution is obtained. The lower brain tissue distribution eliminates the central side effects of yohimbine such as anxiety and increased blood pressure. The compound of the present invention can significantly improve the blood glucose metabolism of animal models of type 2 diabetes. Chronic administration can stabilize the basal blood glucose level of diabetic mice at a level closer to normal, and is expected to be used in the treatment of diabetes.

All documents mentioned in the present invention are cited as references in this application, just as each document is cited as reference individually. In addition, it should be understood that after reading the above teachings of the present invention, those skilled in the art can make various changes or modifications to the present invention, and these equivalent forms also fall within the scope defined by the claims attached to this application.

## Claims

1. A compound represented by general formula (I), a deuterated compound thereof or a pharmaceutically acceptable salt thereof, 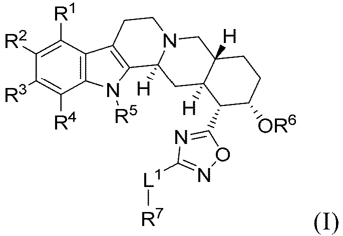 wherein,
R¹, R², R³ and R⁴ are each independently hydrogen, halogen, cyano, hydroxyl, ethynyl, substituted or unsubstituted following group: C1-C6 alkyl, C3-C10 cycloalkyl, 4-7 membered heterocycloalkyl containing 1 to 3 heteroatoms, C6-C10 aryl, 5-7 membered heteroaryl, 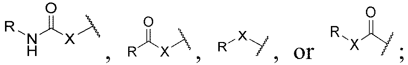
wherein R is C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{a}; wherein R^{a} is selected from hydrogen, C1-C6 alkyl or C3-C10 cycloalkyl;
R⁵ and R⁶ are each independently hydrogen, substituted or unsubstituted following group: C1-C6 alkyl, C3-C10 cycloalkyl, 4-7 membered heterocycloalkyl containing 1 to 3 heteroatoms, 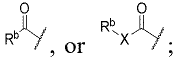 wherein R^{b} is selected from: C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{c}; wherein R^{c} is selected from: hydrogen, C1-C6 alkyl, or C3-C10 cycloalkyl;
L¹ is absent, hydrogen, substituted or unsubstituted following group: C1-C6 alkylene, C3-C10 cycloalkyl, 4-7 membered heterocycloalkyl containing 1 to 3 heteroatoms, C6-C10 aryl, 5-7 membered heteroaryl, 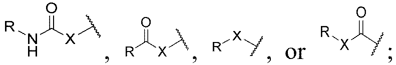 wherein R is C1-C6 alkyl, C3-C10 cycloalkyl, 4-7 membered heterocycloalkyl containing 1 to 3 heteroatoms, C6-C10 aryl, or 5-7 membered heteroaryl, X is O or NR^{a}; wherein R^{a} is selected from: hydrogen, C1-C6 alkyl, or C3-C10 cycloalkyl;
R⁷ is absent, hydrogen, substituted or unsubstituted following group: C1-C6 alkoxy, C3-C10 cycloalkyl, C6-C10 aryl, 3-7 membered heterocyclyl, 5-7 membered heteroaryl, 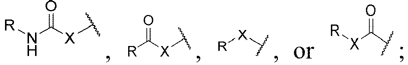
wherein R is C1-C6 alkyl, C3-C10 cycloalkyl, 4-7 membered heterocyclyl containing 1 to 3 heteroatoms, C6-C10 aryl, or 5-7 membered heteroaryl, X is O or NR^{a}; wherein R^{a} is hydrogen, C1-C6 alkyl, or C3-C10 cycloalkyl;
the substituents separated by 0 to 2 atoms on L¹, R⁷ can be cyclized to form 3-7 membered cycloalkyl ring, 4-7membered heterocycloalkyl ring, 6-membered aromatic ring, or 5-7 membered heteroaromatic ring;
the above-mentioned substitution means that a hydrogen on the group is substituted by one or more substituents selected from a group consisting of halogen, hydroxyl, cyano, C1-C6 haloalkyl, alkynyl, C1-C6 alkyl, C1-C6 alkoxy, C1-C6 haloalkoxy, NR^{d}R^{e}, 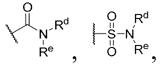 and 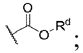 wherein R^{d} and R^{e} are each independently hydrogen or C1-C6 alkyl.

2. The compound of claim 1, wherein R¹ is hydrogen, halogen, cyano, hydroxyl, ethynyl, or C1-C6 alkyl;
R² is hydrogen, halogen, cyano, hydroxyl, ethynyl, C1-C4 alkyl, or 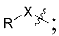 wherein R is a substituted or unsubstituted following group: C1-C4 alkyl, X is O or NR^{a}; wherein R^{a} is hydrogen or C1-C4 alkyl, wherein the substitution means that a hydrogen on the group is substituted by one or more substituents selected from a group consisting of halogen, hydroxyl, C1-C4 alkyl, and NR^{d}R^{e}, wherein R^{d} and R^{e} are each independently hydrogen or C1-C4 alkyl;
R³ is hydrogen, halogen, cyano, hydroxyl, C1-C4 alkyl or 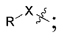 wherein R is a substituted or unsubstituted following group: C1-C4 alkyl, X is O, wherein the substitution means that a hydrogen on the group is substituted by one or more substituents selected from a group consisting of halogen, hydroxyl, cyano, C1-C6 alkyl, and NR^{d}R^{e}, wherein R^{d} and R^{e} are each independently hydrogen, or C1-C4 alkyl;
R⁴ is hydrogen, halogen, cyano, or hydroxyl.

3. The compound of claim 1, wherein R⁵ is hydrogen, C1-C4 alkyl, or 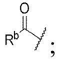 wherein R^{b} is substituted or unsubstituted following group: C1-C4 alkyl, C3-C6 cycloalkyl, C6-C10 aryl, or 5-7 membered heteroaryl, wherein the substitution means that a hydrogen on the group is substituted by one or more substituents selected from a group consisting of halogen, hydroxyl, C1-C4 alkyl, C1-C4 alkoxy, and NR^{d}R^{e}, wherein R^{d} and R^{e} are each independently hydrogen or C1-C4 alkyl;
R⁶ is hydrogen, C1-C4 alkyl, C3-C6 cycloalkyl, or 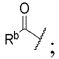 wherein R^{b} is a substituted or unsubstituted following group: C1-C4 alkyl, wherein the substitution means that a hydrogen on the group is substituted by one or more substituents selected from a group consisting of halogen, hydroxyl, cyano, trifluoromethyl, and NR^{d}R^{e}, wherein R^{d} and R^{e} are each independently hydrogen or C1-C4 alkyl.

4. The compound of claim 1, wherein L¹ is absent, hydrogen, substituted or unsubstituted following group: C1-C4 alkylene, C3-C8 cycloalkyl, 5-7 membered heterocycloalkyl containing 1 to 3 heteroatoms, C6-C10 aryl, 5-7 membered heteroaryl, 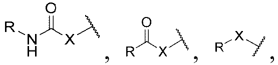 or 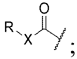 wherein R is C1-C4 alkyl, C3-C6 cycloalkyl, 4-7 membered heterocycloalkyl containing 1 to 3 heteroatoms, C6-C10 aryl, or 5-7 membered heteroaryl, X is O or NR^{a}; wherein R^{a} is hydrogen, C1-C4 alkyl, or C3-C6 cycloalkyl, wherein the substitution means that a hydrogen on the group is substituted by one or more substituents selected from a group consisting of halogen, hydroxyl, cyano, trifluoromethyl, C1-C4 alkyl, C1-C4 alkoxy, and NR^{d}R^{e}, wherein R^{d} and R^{e} are each independently hydrogen or C1-C4 alkyl.

5. The compound of claim 1, wherein R⁷ is absent, hydrogen, substituted or unsubstituted following group: C1-C4 alkoxy, C3-C6 cycloalkyl, C6-C10 aryl, 5-7 membered heterocyclyl, 5-7 membered heteroaryl, 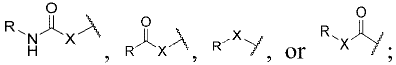 wherein R is C1-C6 alkyl, C3-C10 cycloalkyl, 4-7 membered heterocyclyl containing 1 to 3 heteroatoms, C6-C10 aryl, 5-7 membered heteroaryl, X is O or NR^{a}; wherein R^{a} is hydrogen, C1-C6 alkyl, or C3-C10 cycloalkyl;
the substituents separated by 0 to 2 atoms on R⁷ can be cyclized to form a 3-7 membered cycloalkyl ring, 4-7 membered heterocycloalkyl ring, 6-membered aromatic ring, or 5-7 membered heteroaromatic ring,
wherein the substitution means that a hydrogen on the group is substituted by one or more substituents selected from a group consisting of halogen, hydroxyl, cyano, C1-C4 haloalkyl, C1-C4 alkyl, C1-C4 alkoxy, C1-C4 haloalkoxy, NR^{d}R^{e}, 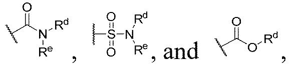 wherein R^{d} and R^{e} are each independently hydrogen or C1-C4 alkyl;
preferably, R⁷ is absent, hydrogen, substituted or unsubstituted following group: C1-C4 alkoxy, C3-C6 cycloalkyl, phenyl, 5-7 membered heterocyclyl, or 5-7 membered heteroaryl;
wherein the substitution means that a hydrogen on the group is substituted by one or more substituents selected from a group consisting of fluorine, chlorine, bromine, hydroxyl, cyano, trifluoromethyl, trifluoromethoxy, alkynyl, C1-C4 alkyl, C1-C4 alkoxy, NR^{d}R^{e}, 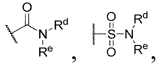 and 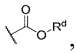 wherein R^{d} and R^{e} are each independently hydrogen or C1-C4 alkyl;
the substituents separated by 0 to 2 atoms on R⁷ can be cyclized to form a 6-membered aromatic ring or a 5-7 membered heteroaromatic ring.

6. The compound of claim 1, wherein L¹ and R⁷ form the following formula V: 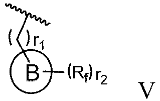
wherein r₁ is selected from 1, 2 or 3; r₂ is selected from 0, 1, 2 or 3;
ring B is phenyl, 5-6 membered heterocyclyl, C3-C6 cycloalkyl, or 5-6 membered heteroaryl;
each R_{f} is independently selected from: halogen, hydroxyl, cyano, C1-C4 haloalkyl, C1-C4 alkyl, C1-C4 alkoxy, C1-C4 haloalkoxy, NR^{d}R^{e}, 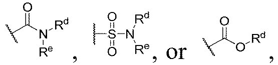 wherein R^{d} and R^{e} are each independently hydrogen or C1-C4 alkyl; or two adjacent R^{f} and the adjacent C on ring B together form a benzene ring or a 5-7 membered heteroaromatic ring.

7. The compound of claim 1, wherein the compound is selected from:

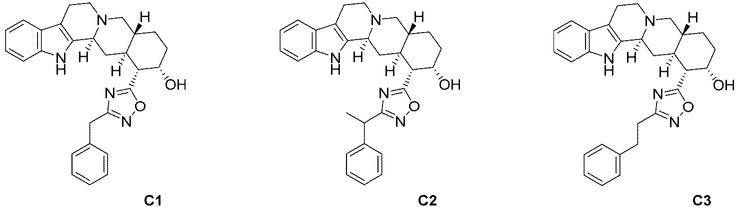

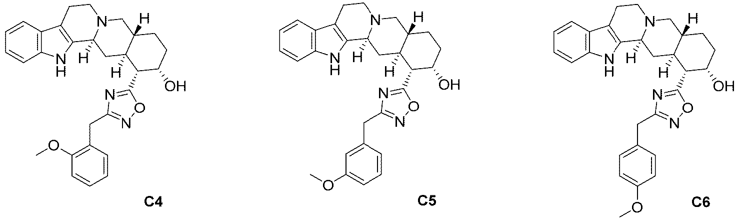

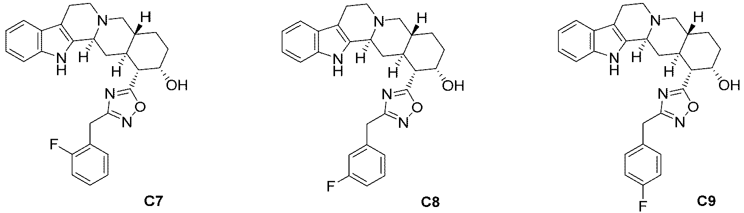

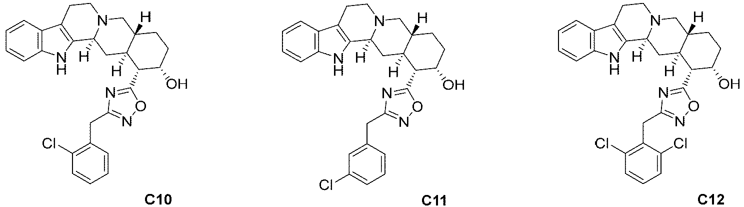

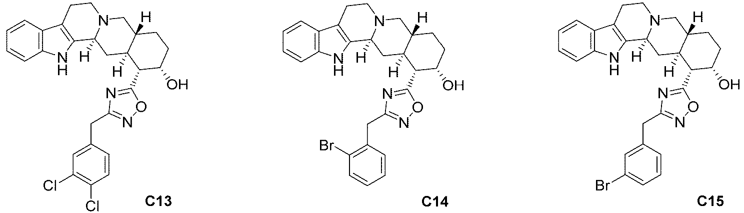

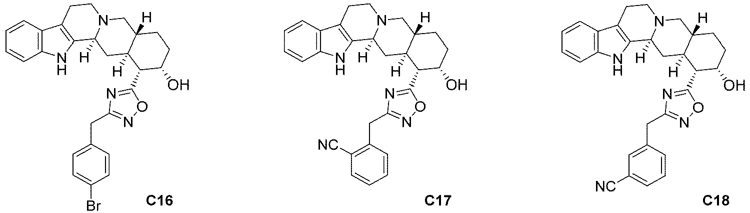

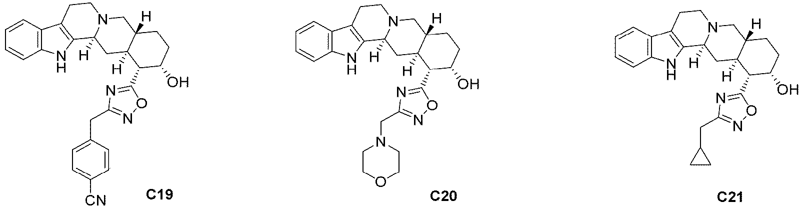

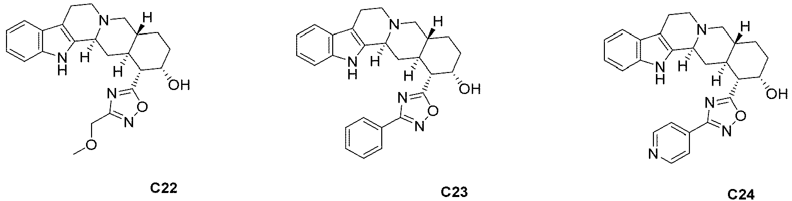

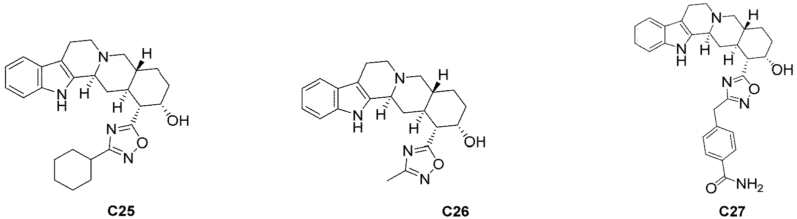

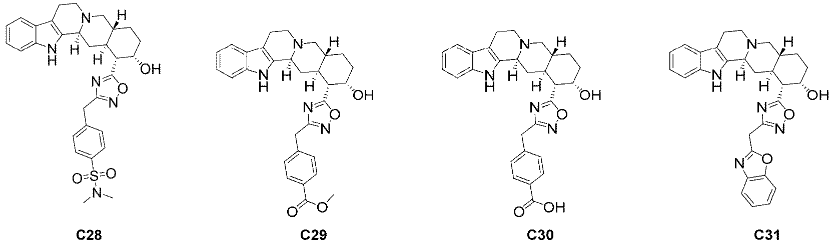

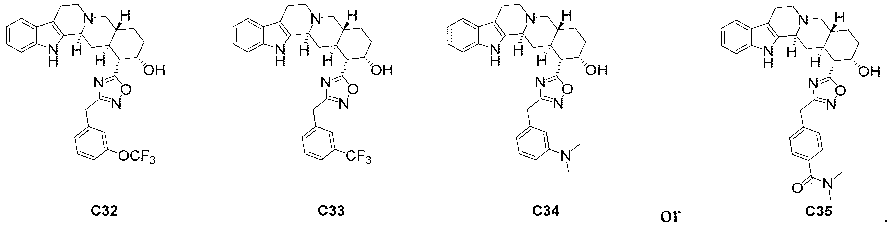

8. A method for preparing the compound of claim 1, wherein in the method, 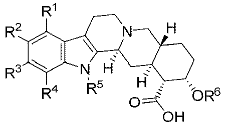 is used as a raw material, and the compound is obtained by converting the 16-carboxyl group into an oxadiazole structure, wherein each substituent is as defined in claim 1.

9. A pharmaceutical composition comprising:
the compound represented by the general formula (I) of claim 1, or a pharmaceutically acceptable salt thereof; and
a pharmaceutically acceptable carrier.

10. Use of the compound represented by the general formula (I) of claim 1 or the pharmaceutical composition of claim 9 for (i) the manufacture of an α2A adrenergic receptor (α2A-AR) antagonist; or (ii) the manufacture of a drug for treating diabetes.
